# EUROPEAN PATENT SPECIFICATION

(11) **EP 4 686 494 B1**
(45) Date of publication and mention of the grant of the patent: **28.10.2026**
(21) Application number: 25188645.3
(22) Date of filing: 10.07.2025
(51) Int. Cl.: A61N 1/372, A61N 1/375

(54) **IMPROVED CONDUCTIVE IMPLANT TO IMPLANT COMMUNICATION FOR USE WITH IMPLANTABLE MEDICAL DEVICES**
VERBESSERTES LEITFÄHIGES IMPLANTAT ZUR IMPLANTATKOMMUNIKATION ZUR VERWENDUNG MIT IMPLANTIERBAREN MEDIZINISCHEN VORRICHTUNGEN
COMMUNICATION AMÉLIORÉE D'IMPLANT CONDUCTEUR À IMPLANT DESTINÉE À ÊTRE UTILISÉE AVEC DES DISPOSITIFS MÉDICAUX IMPLANTABLES

(30) Priority: 01.08.2024 US 202463678231 P; 17.06.2025 US 202519241100
(43) Date of publication of application: 04.02.2026
(73) Proprietor: Pacesetter, Inc., Sylmar, CA 91342 (US)
(72) Inventor: Xin, Xiyao, Sylmar, CA 91342 (US); Sison, Shiloh, Sylmar, CA 91342 (US); Chen, Xi Lin, Sylmar, CA 91342 (US)
(74) Representative: Barker Brettell Sweden AB

(56) References cited:
- EP-B1- 3 589 363
- WO-A1-2020/264108
- US-B2- 8 050 297

## Description

### FIELD OF TECHNOLOGY

Embodiments described herein generally relate to methods, devices, and systems for providing and improving communication between implantable medical devices, one or more of which may be a leadless pacemaker.

### BACKGROUND

Implantable medical devices (IMDs) and systems often rely on proper communication to operate correctly. For example, in a multi-chamber (e.g., dual chamber) leadless pacemaker system, implant-to-implant (i2i) communication between two or more leadless pacemakers (LPs) is used for proper synchronization and operation of the system. Conductive communication involves transmitting and receiving communication signals through patient tissue, typically (but not necessarily) using the same electrodes that are used for cardiac pacing and sensing, wherein the transmitted communication signals include pulses that can be produced using the same pulse generator that is used (or a different pulse generator than is used) to produce pacing pulses. Conductive communication, which is also known as conducted communication, or galvanic communication, is much more energy efficient than radio frequency (RF) communication. Accordingly, the longevity of a multi-chamber LP system that utilizes conducted communication for i2i communication is greater than the longevity of a multi-chamber LP system that utilizes RF communication for i2i communication. Where conducted communication is used for i2i communication, the communication can be referred to as conductive i2i communication. Conductive i2i communication, as with any communication modality, is susceptible to disruptions due to extrinsic interference (aka extrinsic noise).

US 8050297 B2 discloses a system and method that facilitates multiple systems of communicating devices to coexist on a common communication channel having a limited temporal bandwidth while maintaining the required update rate between each master device and its associated slave devices. The master devices periodically transmit one or more beacon messages that are suitable for identification by other such master devices at a communication range greater than the communication range that may cause interference between systems and thus enabling one or more systems to cause the position of its frame periods to be interleaved with the frame periods of other such systems in anticipation of systems moving in closer proximity and actually interfering with each other.

EP 3589363 B1 discloses systems, apparatus, methods and computer-readable storage media that mitigate IMD power drain associated with stalled telemetry sessions are provided. A device is configured to receive a communication request from an external device via a telemetry communication link established between the IMD and the external device. The communication request can comprise a request for data. The device can further comprise a throughput monitoring component configured to monitor uplink throughput associated with transmission of the data by the IMD to the external device via the telemetry communication link based on reception of the communication request. The communication component is configured to terminate the telemetry communication link based on the uplink throughput being below a threshold value.

WO 2020264108 A1 discloses systems, devices, and methods for validating a position of a wirelessly powered electrostimulation device while the device is implanted in body tissue. A method can include situating the electrostimulation device in tissue and before an affixation mechanism of the electrostimulation device is deployed to maintain an implanted position of the electrostimulation device, and while electrodes of the device are in contact with the tissue, performing electrical testing of the electrostimulation device to determine whether the electrostimulation from the electrostimulation device evokes a specified response from the body that contains the tissue.

### SUMMARY

The invention is defined in the appended claims. Aspects, embodiments and examples disclosed herein which do not fall within the scope of the appended claims do not form part of the invention, and are merely provided for illustrative purposes.

Certain embodiments of the present technology relate to an IMD configured to communicate using conductive i2i communication with another IMD that is remotely located relative to the IMD. The IMD comprises a plurality of electrodes, a conductive communication transceiver, and a controller. The conductive communication transceiver is coupled to at least two of the plurality of electrodes and configured to selectively transmit outgoing conductive i2i communication messages that are intended for the other IMD, and configured to receive conductive i2i communication messages from the other IMD. The controller is operatively coupled to the conductive communication transceiver and configured to control when the IMD is in a first conductive i2i communication mode and when the IMD is in a second conductive i2i communication mode. The controller is further configured to control the conductive communication transceiver to transmit outgoing conductive i2i communication messages that includes event markers and to attempt to receive valid incoming conductive i2i communication messages that include event markers from the other IMD while the IMD is in the first conductive i2i communication mode. The controller is also configured to detect when a quantity of possible incoming conductive i2i communication messages that are determined to be invalid during a first specified period reach an invalid message count threshold while the IMD is in the first conductive i2i communication mode, and in response thereto, cause a switch from the first conductive i2i communication mode to the second conductive i2i communication mode. Additionally, the controller is configured to control, while the IMD is in the second conductive i2i communication mode, the conductive communication transceiver to transmit outgoing conductive i2i beacon messages to the other IMD less frequently than the outgoing conductive i2i communication messages transmitted when the IMD is in the first conductive i2i communication mode.

In accordance with certain embodiments, the controller is further configured to perform a message validation operation on each possible incoming conductive i2i communication message of one or more possible incoming conductive i2i communication messages received during a first specified period while the IMD is in the first conductive i2i communication mode, and to monitor a quantity of possible incoming conductive i2i communication messages that are determined to be invalid during the first specified period while the IMD is in the first conductive i2i communication mode. The controller is further configured to control the conductive communication transceiver to transmit outgoing conductive i2i beacon messages to the other IMD once every second specified period while the IMD is in the second conductive i2i communication mode, wherein that the second specified period is longer than the first specified period, and to attempt to receive valid incoming conductive i2i beacon messages from the other IMD, wherein each said outgoing conductive i2i beacon message that the IMD transmits is devoid of an event marker, and wherein each said valid incoming conductive i2i beacon message that the IMD attempts to receive is devoid of an event marker. The controller is further configured to detect when a said valid incoming conductive i2i beacon message has been received while the IMD is in the second conductive i2i communication mode or determine that the IMD has been in the second conductive i2i communication mode for a third specified period, and based thereon, cause a switch from the second conductive i2i communication mode back to the first conductive i2i communication mode, wherein the third specified period is longer than the second specified period.

Certain embodiments of the present technology relate to a system including the IMD for which various embodiments of thereof are summarized above, and the other IMD that is remotely located related to the IMD.

In certain embodiments, at least one of the IMD and the other IMD is an LP.

In certain such embodiments, the IMD is a first LP and the other IMD is a second LP.

In certain embodiments, at least one of the IMD and the other IMD is an ICD or an ICM.

Certain embodiments of the present technology related to methods for use by an IMD that communicates with another IMD using conductive i2i communication, wherein such methods can be used to improve the i2i communication between the IMDs. In accordance with certain embodiments, the method includes while in a first conductive i2i communication mode, the IMD transmitting outgoing conductive i2i communication messages that includes event markers to the other IMD, and the IMD attempting to receive valid incoming conductive i2i communication messages that includes event markers from the other IMD. The method also includes detecting when a quantity of possible incoming conductive i2i communication messages that are determined to be invalid during a first period reach an invalid message count threshold while the IMD is in the first conductive i2i communication mode, and in response thereto, switching from the first conductive i2i communication mode to a second conductive i2i communication mode that consumes on average less power per cardiac cycle than the first conductive i2i communication mode. The method additionally includes while the IMD is in the second conductive i2i communication mode, transmitting outgoing conductive i2i beacon messages to the other IMD less frequently than the outgoing conductive i2i communication messages are transmitted when the IMD is in the first conductive i2i communication mode.

In accordance with an embodiment, the method comprises while in the first conductive i2i communication mode and while attempting to receive valid incoming conductive i2i communication messages from the other IMD during, the IMD performing a message validation operation on one or more possible incoming conductive i2i communication messages received during a first specified period, and the IMD monitoring a quantity of possible incoming conductive i2i communication messages that are determined to be invalid during the first specified period. Additionally, the method comprises the IMD detecting when the quantity of possible incoming conductive i2i communication messages that are determined to be invalid during the first specified period reach an invalid message count threshold, and in response thereto the IMD switching from the first conductive i2i communication mode to a second conductive i2i communication mode that consumes on average less power per cardiac cycle than the first conductive i2i communication mode. Additionally, the method comprises while in the second conductive i2i communication mode, the IMD transmitting an outgoing conductive i2i beacon message to the other IMD once every second specified period, wherein the second specified period is longer the first specified period, and the IMD attempting to receive valid incoming conductive i2i beacon messages from the other IMD, wherein each said outgoing conductive i2i beacon message that the IMD transmits is devoid of an event marker, and wherein each said valid incoming conductive i2i beacon message that the IMD attempts to receive is devoid of an event marker. Additionally, the method comprises while in the second conductive i2i communication mode, the IMD detecting a said valid incoming conductive i2i beacon message or the IMD determining that the IMD has been in the second conductive i2i communication mode for a third specified period, and based thereon the IMD switching from the second conductive i2i communication mode back to the first conductive i2i communication mode, wherein the third specified period is longer than the second specified period.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

### BRIEF DESCRIPTION OF THE DRAWINGS

Embodiments of the present technology relating to both structure and method of operation may best be understood by referring to the following description and accompanying drawings, in which similar reference characters denote similar elements throughout the several views:
FIG. 1 illustrates a system formed in accordance with certain embodiments herein as implanted in a heart.
FIG. 2 is a block diagram of a single LP in accordance with certain embodiments herein.
FIG. 3 illustrates an LP in accordance with certain embodiments herein.
FIG. 4 is a timing diagram demonstrating one embodiment of i2i communication for a paced event.
FIG. 5 is a timing diagram demonstrating one embodiment of i2i communication for a sensed event.
FIG. 6 is a high level flow diagram that is used to summarize methods for improving communication between IMDs, according to certain embodiments of the present technology.
FIG. 7A illustrates example contents of a conductive i2i communication message, according to certain embodiment of the present technology.
FIG. 7B illustrates example contents of a conductive i2i beacon message, according to certain embodiment of the present technology.
FIG. 8 shows a block diagram of one embodiment of an IMD (e.g., LP or in ICD) that is implanted into the patient as part of an implantable cardiac system in accordance with certain embodiments herein.

### DETAILED DESCRIPTION

Certain embodiments of the present technology related to implantable medical devices (IMDs), and methods for use therewith, that improve implant-to-implant (i2i) communication between the IMDs, one or more of which may be a leadless pacemaker (LP).

As noted above, conductive i2i communication, as with any communication modality, is susceptible to disruptions due to extrinsic interference (aka extrinsic noise). In a multi-chamber leadless pacemaker (LP) system of an embodiment of the present technology, such as a dual chamber LP system, whenever an LP receives pulses that could potentially be a conductive i2i communication message (and more generally, a conductive i2i packet), the LP that receives the potential (aka possible) incoming conductive i2i communication message attempts to decode the possible message and determines whether it is a valid message. A received possible incoming conductive i2i communication message is determined to be valid if the message as decoded includes a valid code (sequence of bits), that can be one of one or more (e.g., four) predetermined codes that is recognizable to the LP (or other type of IMD) that received the message. Otherwise the received message is determined to be invalid, and is ignored. When an LP (or other type of IMD) detects an invalid i2i message, it could be said that the LP detects an i2i Null. If the received possible incoming conductive i2i communication message is determined to be valid it can be used by the LP that received the message, e.g., to time when to perform pacing or other therapy, to perform a command included in the received message, and/or the like. The above described operation that an LP performs can be referred to herein as a message validation operation. In accordance with certain embodiments of the present technology, an LP performs this message validation operation each time the LP receives what appears to be a wakeup pulse that should precede a conductive i2i communication message transmitted by another LP. When extrinsic interference is not present or is relatively low, an LP will typically only receive one conductive i2i communication message, and thus typically only one wakeup pulse, from another LP per cardiac cycle. However, when extrinsic interference is relatively high, an LP may receive numerous pulses that each appear to be a wakeup pulse but are actually just noise. Accordingly, when extrinsic interference is relatively high, an LP may perform the above described message validation operation numerous times per cardiac cycle, because the message validation operation is performed in response to each received pulse that appears to be a wakeup pulse. In accordance with certain embodiments, a received pulse may appear to be a wakeup pulse if energy of the received pulse within a specified frequency range (e.g., a passband from 1 kHz through 100 kHz, but not limited thereto) exceeds a wakeup pulse detection threshold. The aforementioned extrinsic interference can be, for example, power line noise, electromagnetic interference (EMI), or electromyogenic artifacts, but is not limited thereto. The term "possible" is used in the phrase "possible incoming conductive i2i communication message" to clarify that such an incoming message may not be an actual valid message, but rather, may be extrinsic interference that is distinguishable from an actual valid message, e.g., using the message validation operation.

In accordance with certain embodiments of the present technology, as another layer of protection on top of the aforementioned packet validation that an LP performs on each received possible incoming conductive i2i communication message, and more specifically in response to each received pulse that appears to be a wakeup pulse, the LP is also configured to identify the presence of persistent interference (aka persistent noise) within the passband of at least one of its one or more conductive i2i communication receiver(s). If such persistent i2i interference is detected, the receiving LP may be configured to temporarily disable its reception and transmission of i2i communications for a disable period (e.g., a specified duration of time, or a specified number of cardiac cycles) before again checking to determine whether the i2i interference is still present. This mitigation feature within an implantable multi-chamber (e.g., dual chamber) LP system that utilizes conductive i2i communication can be referred to "i2i Noise Reversion" and is intended to further reduce the possibility of inadvertent false-positive or false-negative errors occurring during i2i packet validation, while also conserving power. While the LP is in its i2i Noise Reversion mode, which can also be referred to as a Noise State, the LP can operate in a safe pacing mode (e.g., VVI or VOO, or AAI or AOO) that does not depend on i2i communication.

More specifically, an LP may transition to its i2i Noise Reversion Mode (aka Noise State) if a threshold number Q of invalid messages are received within a specified period P. The specified period P during (during which if the LP receives the threshold number Q of invalid message, the LP will transition to its i2i Noise Reversion Mode) can be one cardiac cycle, a specified number of consecutive cardiac cycles (e.g., within the range of 2 to 5 cardiac cycles, but not limited thereto), or a specified duration of time (e.g., within the range of 1 to 5 seconds, but not limited thereto). The threshold number Q can be programmable within a specified range (e.g., from 1 to 30), and may have a default value (e.g., 10 or 15). Explained another way, the LP transitions to its i2i Noise Reversion Mode (aka Noise State) if a threshold number Q of i2i Nulls are detected within a specified period P (e.g. one cardiac cycle, a specified number of consecutive cardiac cycles, or specified duration of time). As mentioned above, once an LP enters is its i2i Noise Reversion Mode (aka Noise State) the LP may remain in that state for a disable period, during which it may operate in a safe pacing mode. The disable period can be programmable. For example, the disable period can be a number of consecutive cardiac cycles within a specified range (e.g., from 1 to 255 consecutive cardiac cycles), and may have a default value (e.g., 64). The disable period can alternatively be a specified duration of time, e.g., from 1 to 255 seconds, and may have a default value, e.g., 64 seconds. At the end of the disable period, the LP enables (at least temporarily) its reception and transmission of i2i communications, to determine whether there is a continued presence of i2i interference. If so, then the LP will reenter its Noise Reversion Mode (aka Noise State) for another disable period, before checking again to determine whether there is a continued presence of i2i interference. If not, i.e., once the presence of i2i interference is no longer detected, the LP will keep its reception and transmission of i2i communications enabled. It is noted that where a specified period is defined as being equal to a specified number of cardiac cycles, the length of the specified period will change if the length of the cardiac cycles change. For example, a period of five cardiac cycles that are each 800 msec in length is longer than a period of five cardiac cycles that are each 500 msec in length (i.e., 5 x 800 msec > 5 x 500 msec).

While an LP is in the i2i Noise Reversion Mode, beat-to-beat AV synchrony may not be maintained because the aLP and the vLP are not communicating with one another on a beat-by-beat basis. Accordingly, if the disable period is programmed to be relatively long, e.g., 255 cardiac cycles, then once the LPs enter the i2i Noise Reversion mode the LPs will remain in the i2i Noise Reversion mode for at least 255 cardiac cycles, during which beat-to-beat AV synchrony may not be maintained, which is undesirable.

Certain embodiments of the present technology are directed to modifications to the above summarized i2i Noise Reversion mode, whereby an LP that is in its i2i Noise Reversion mode performs checks, from time-to-time (e.g., once per N cardiac cycles, or once per N seconds), of whether extrinsic interference has subsided such that the LP could exit the i2i Noise Reversion mode earlier than it otherwise would, i.e., without waiting until the end of the disable period (e.g., without waiting 255 cardiac cycles, or some programmed number of cardiac cycles or some programmed duration of time). This modified i2i Noise Reversion mode can also be referred to herein as an "i2i Noise Reversion with checking" mode. In contrast to the existing i2i Noise Reversion mode (in which i2i transmission and receiving capabilities are disabled during an entirety of the disable period when an LP is in its i2i Noise Reversion mode), in the i2i Noise Reversion with checking mode of certain embodiments of the present technology, the i2i transmission and receiving capabilities are still enabled (at least part of the time) when an LP is in that mode, but the i2i transmission and receiving capabilities are used sparingly (to conserve power) by an LP solely to determine whether the LP can exit the i2i Noise Reversion mode early because the noise (that had caused the LP to enter the i2i Noise Reversion mode) is no longer present or has sufficiently subsided such that it no longer prevents receiving of valid conductive i2i messages.

In accordance with certain embodiments of the present technology, an LP may enter the "i2i Noise Reversion with checking" mode in the same manner as an LP enters the above described i2i Noise Reversion mode. More specifically, in accordance with certain embodiments, an LP will enter the "i2i Noise Reversion with checking" mode if a threshold number Q of invalid messages are received within a specified period P (e.g. one cardiac cycle, a specified number of consecutive cardiac cycles, or specified duration of time), where the threshold number Q can be programmable within a specified range (e.g., from 1 to 30), and may have a default value (e.g., 10 or 15). Explained another way, an LP may transition to its "i2i Noise Reversion with checking" mode if a threshold number Q of i2i Nulls are detected within a specified period P (e.g. one cardiac cycle, a specified number of consecutive cardiac cycles, or specified duration of time). While in the "i2i Noise Reversion with checking" mode an LP will transmit an i2i beacon message (which is devoid of an event marker) once every N cardiac cycles (where N is programmable). In certain embodiments, the i2i beacon message is preceded by a wakeup pulse, and includes a predetermined value (known by the other LP) followed by a cyclic redundancy check (CRC) code (or some other error detection code). In contrast to the more typical conductive i2i communication message, the i2i beacon message does not include any event marker and does not include any command. Similar to the more typical conductive i2i communication message, the conductive i2i beacon message is transmitted by the sending LP just prior to (or just after) a paced event, or just after a sensed event. In accordance with certain embodiments of the present technology, in addition to each LP transmitting the i2i beacon message from time-to-time (while each LP is in its i2i Noise Reversion with checking mode), each LP also attempts to receive an i2i beacon message using its receiver(s). When the LP receives what appears to be the i2i beacon message it decodes the message and determines whether the value therein matches the predetermined value and whether the CRC code (or other error detection code) is correct. If the LP successfully receives a valid conductive i2i beacon message then the LP assumes that the extrinsic interference has subsided and the LP exits the "i2i Noise Reversion with checking" mode. Alternatively, the LP may need to receive a specified/programmed plurality of valid i2i beacon messages before the LP assumes that the extrinsic interference has subsided and the LP exits the "i2i Noise Reversion with checking" mode. For example, in accordance with certain embodiments, an LP must receive a specified plurality X of valid i2i beacon messages, or must receive valid i2i beacon messages in X out of Y consecutive cardiac cycles, or must receive a specified plurality X of valid i2i beacon messages within a specified duration of time, in order for the LP to exit early out of the "i2i Noise Reversion with checking" mode. Once the LP has exited early out of the "i2i Noise Reversion with checking" mode, the LP will return to its normal conductive i2i communication mode.

In accordance with certain embodiments, each LP (e.g., aLP and vLP) of a multi-chamber LP system transmits the same i2i beacon message while the LPs are in the "i2i Noise Reversion with checking" mode. In accordance with other embodiments, each LP (e.g., aLP and vLP) of a multi-chamber LP system transmits its own unique i2i beacon message that differs from the i2i beacon message transmitted by the other LP(s) of the multi-chamber LP system, e.g., the i2i beacon message transmitted by an aLP differs from that transmitted by a vLP. Either embodiment will work, so long as the LP that receives the i2i beacon message from another LP knows the predetermined value of the i2i beacon message transmitted by the other LP.

In accordance with certain embodiments, if only one of an aLP and a vLP has exited its i2i noise reversion with checking mode, the existing handshaking attempts will still be made until both devices have exited i2i Noise Reversion with checking mode allowing the dual chamber communication.

The i2i Noise Reversion with checking mode introduced above was generally described as being used by at least two LPs of a multi-chamber (e.g., dual chamber) LP system. However, as will be appreciated by the description below, the i2i Noise Reversion with checking mode can be used by other types of IMDs that are configured to perform conductive i2i communication besides and/or in addition to LPs. Examples of other types of IMDs that can be configured to use i2i Noise Reversion with checking mode include an implantable cardioverter-defibrillators (ICD), and an insertable cardiac monitors (ICM), but are not limited thereto.

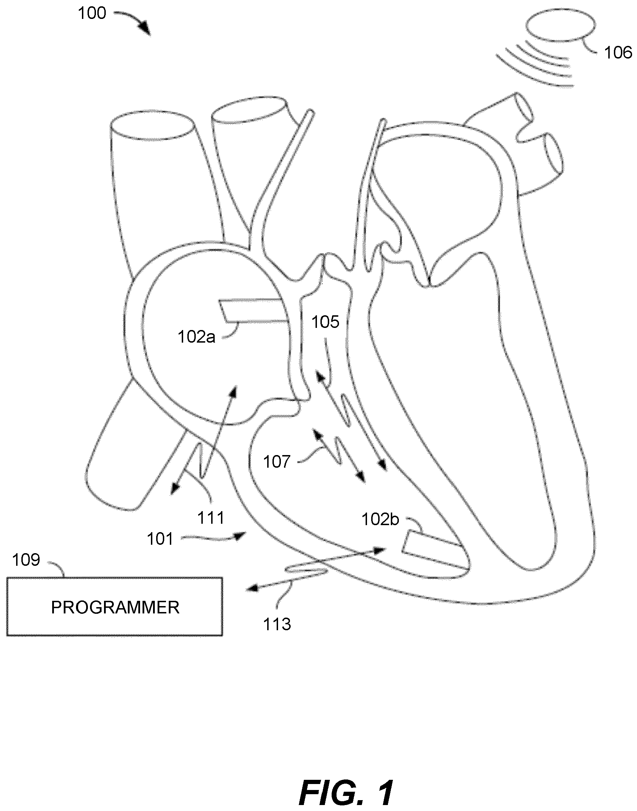

Before providing additional details of the specific embodiments of the present technology mentioned above, an example system in which embodiments of the present technology can be used will first be described with reference to FIGS. 1-5. More specifically, FIGS. 1-5 will be used to describe an example cardiac pacing system, wherein pacing and sensing operations can be performed by multiple medical devices, which may include one or more leadless cardiac pacemakers, a non-vascular ICD, such as a subcutaneous-ICD (S-ICD), and/or a programmer to reliably and safely coordinate pacing and/or sensing operations. A leadless cardiac pacemaker can also be referred to more succinctly herein as a leadless pacemaker (LP). Where a cardiac pacing system includes a non-vascular ICD (e.g., an S-ICD), the non-vascular ICD may perform certain sensing operations and may communicate with one or more LPs by sending and/or receiving messages to and/or from one or more LPs, as can be appreciated from the below discussion. Where a cardiac pacing system includes a programmer, the programmer may be used to program one or more IMDs, download information to one or more IMDs, and/or upload information from one or more IMDs, as can be appreciated from the below description.

FIG. 1 illustrates an example of a system 100 that is configured to be implanted in a heart 101. The system 100 includes two or more leadless pacemakers (LPs) 102a and 102b located in different chambers of the heart. LP 102a is located in a right atrium, while LP 102b is located in a right ventricle. LPs 102a and 102b communicate with one another to inform one another of various local physiologic activities, such as local intrinsic events, local paced events and the like. LPs 102a and 102b may be constructed in a similar manner, but operate differently based upon which chamber the LP 102a or 102b is located. The LPs 102a and 102b may sometimes be referred to collectively herein as the LPs 102, or individually as an LP 102.

In certain embodiments, LPs 102a and 102b communicate with one another, and/or with an ICD 106, by conductive communication, preferably (but not necessarily) through the same electrodes that are used for sensing and/or delivery of pacing therapy. The LPs 102a and 102b may also be able to use conductive communication to communicate with an external device, e.g., a programmer 109, having electrodes placed on the skin of a patient within with the LPs 102a and 102b are implanted. The LPs 102a and 102b can each also include an antenna that would enable them to communicate with one another, the ICD 106 and/or an external device using RF communication. While only two LPs 102a and 102b are shown in FIG. 1, it is possible that the system 100 includes more than two LPs implanted in a patient. For example, to provide for bi-ventricular pacing and/or cardiac resynchronization therapy (CRT), in addition to having LPs 102a and 102b implanted in the right atrial (RA) chamber and the right ventricular (RV) chamber, a further LP can be implanted in the left ventricular (LV) chamber.

In some embodiments, one or more LP 102 can be co-implanted with the ICD 106. Each LP 102 uses two or more electrodes located within, on, or within a few centimeters of the housing of the LP 102, for pacing and sensing at the cardiac chamber, for bidirectional conductive communication with one another, with the programmer 109, and the ICD 106.

While the methods, devices and systems described herein include examples primarily in the context of LPs, it is understood that the methods, devices and systems described herein may be utilized with various other types of IMDs. In other words, the methods, devices and systems may be used to improve communication between various IMDs implanted in a human, not just LPs. For examples, such embodiments can be used to improve communications between at least two LPs, between an LP and an NV-ICD, between an LP and an ICM, between an ICM and an S-ICD, but are not limited thereto. It should also be understood that the embodiments described herein can be used to improve communication between more than two IMDs, and are not limited to communication between just first and second IMDs. The methods, devices and systems may also be used to improve communication between two or more IMDs implanted within the same cardiac chamber that may be the same type of IMD or may be different types of IMDs. The methods, devices and systems may also be used to improve communication between two or more IMDs in a system including at least one IMD that is not implanted within a cardiac chamber, but rather, is implanted epicardially, transmurally, intravascularly (e.g., coronary sinus), or subcutaneously (e.g., S-ICD), etc.

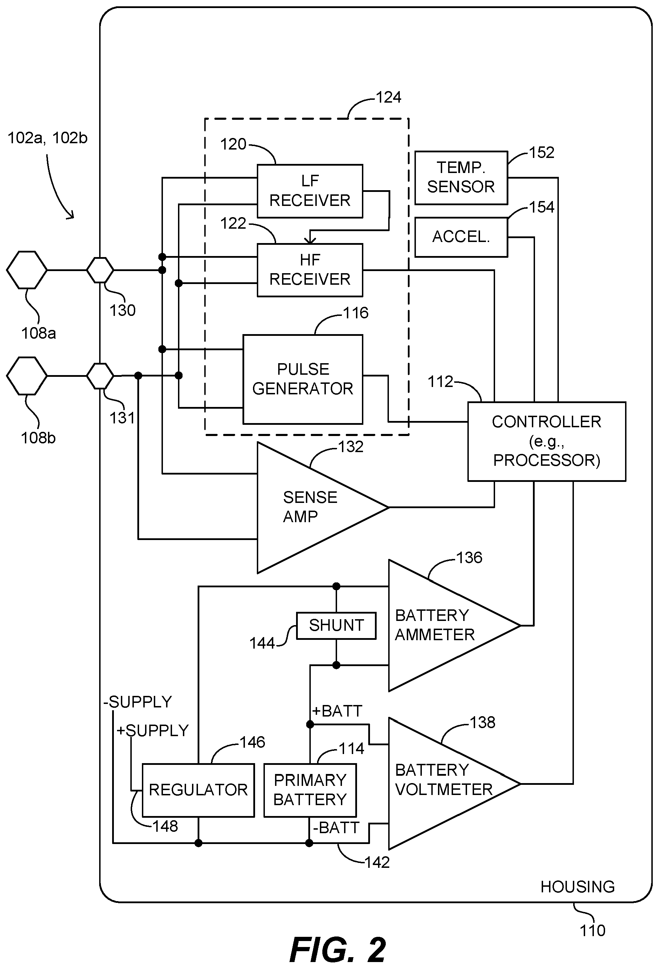

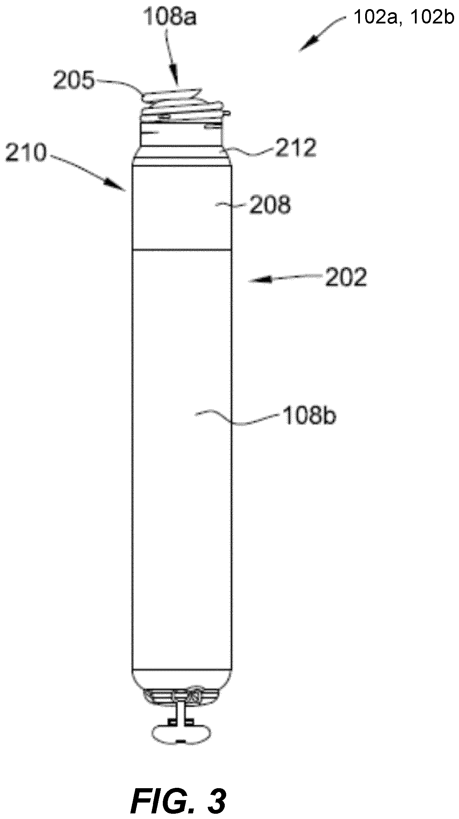

Referring to FIG. 2, a block diagram shows an embodiment for portions of the electronics within LPs 102a, 102b configured to provide conductive communication through the sensing/pacing electrode. One or more of LPs 102a, 102b include at least two leadless electrodes 108a, 108b configured for delivering cardiac pacing pulses, sensing evoked and/or natural cardiac electrical signals, and uni-directional or bidirectional conductive communication. In FIG. 2 (and FIG. 3) the two electrodes shown therein are labeled 108a and 108b. Such electrodes can be referred to collectively as the electrodes 108, or individually as an electrode 108. An LP 102, or other type of IMD, can include more than two electrodes 108, depending upon implementation.

In FIG. 2, each of the LPs 102a, 102b is shown as including first and second receivers 120 and 122 that collectively define separate first and second conductive communication channels 105 and 107 (FIG. 1), (among other things) between LPs 102a and 102b. Although first and second receivers 120 and 122 are depicted, in other embodiments, each LP 102a, 102b may only include the first receiver 120, or more generally may include only a single receiver that is configured to receive conductive communication signals. It is also possible that an LP 102 may include additional receivers other than first and second receivers 120 and 122. As will be described in additional detail below, the pulse generator 116 can function as a transmitter that transmits i2i communication signals using the electrodes 108. In certain embodiments, LPs 102a, 102b may communicate over more than just first and second conductive communication channels 105 and 107. In certain embodiments, LPs 102a, 102b may communicate over one common communication channel 105. More specifically, LPs 102a, 102b can communicate conductively over a common physical channel via the same electrodes 108 that are also used to deliver pacing pulses.

The receivers 120, 122 can also be referred to, respectively, as a low frequency (LF) receiver 120 and a high frequency (HF) receiver 122, because the receiver 120 is configured to monitor for one or more signals within a relatively low frequency range (e.g., below 100 kHz) and the receiver 122 is configured to monitor for one or more signals within a relatively high frequency range (e.g., above 100 kHz). In certain embodiments, the receiver 120 (and more specifically, at least a portion thereof) is always enabled and monitoring for a wakeup notice, which can simply be a wakeup pulse, within a specific low frequency range (e.g., between 1 kHz and 100 kHz); and the receiver 122 is selectively enabled by the receiver 120. The receiver 120 is configured to consume less power than the receiver 122 when both the first and second receivers are enabled. Accordingly, the receiver 120 can also be referred to as a low power receiver 120, and the receiver 122 can also be referred to as a high power receiver 122. The low power receiver 120 is incapable of receiving signals within the relatively high frequency range (e.g., above 100 kHz), but consumes significantly less power than the high power receiver 122. This way the low power receiver 120 is capable of always monitoring for a wakeup notice without significantly depleting the battery 114 of the LP 102a, 102b. In accordance with certain embodiments, the high power receiver 122 is selectively enabled by the low power receiver 120, in response to the low power receiver 120 receiving a wakeup notice, so that the high power receiver 122 can receive the higher frequency signals, and thereby handle higher data throughput needed for effective i2i communication without unnecessarily and rapidly depleting the battery 114 of the LP 102a, 102b (which the high power receiver 122 may do if it were always enabled).

Since the receivers 120, 122 are used to receive conductive communication messages, the receivers 120, 122 can also be referred to as conductive communication receivers. In certain embodiments, each of the LPs 102 includes only a single conductive communication receiver.

In accordance with certain embodiments, when one of the LPs 102a, 102b senses an intrinsic event or delivers a paced event, the corresponding LP 102a, 102b transmits an implant event message to the other LP 102a, 102b. For example, when an aLP 102a senses/paces an atrial event, the aLP 102a transmits an implant event message including an event marker indicative of a nature of the event (e.g., intrinsic/sensed atrial event, paced atrial event). When a vLP 102b senses/paces a ventricular event, the vLP 102b transmits an implant event message including an event marker indicative of a nature of the event (e.g., intrinsic/sensed ventricular event, paced ventricular event). In certain embodiments, each LP 102a, 102b transmits an implant event message to the other LP 102a, 102b preceding the actual pace pulse so that the remote LP c102a, 102b an blank its sense inputs in anticipation of that remote pace pulse (to prevent inappropriate crosstalk sensing). The above describe implant event messages are examples of i2i messages.

The implant event messages may be formatted in various manners. As one example, each event message may include a leading trigger pulse (also referred to as an LP wakeup notice, wakeup pulse or wakeup signal) followed by an event marker. The notice trigger pulse (also referred to as the wakeup notice, wakeup pulse or wakeup signal) is transmitted over a first channel (e.g., with a pulse duration of approximately 10 µs to approximately 1 ms and/or within a fundamental frequency range of approximately 1 kHz to approximately 100 kHz). The notice trigger pulse indicates that an event marker is about to be transmitted over a second channel (e.g., within a higher frequency range). The event marker can then be transmitted over the second channel. More generally, an implant-to-implant (i2i) packet includes a wakeup pulse following by an i2i message.

The event markers may include data indicative of one or more events (e.g., a sensed intrinsic atrial activation for an aLP 102a, a sensed intrinsic ventricular activation for a vLP 102b). The event markers may include different markers for intrinsic and paced events. The event markers may also indicate start or end times for timers (e.g., an AV interval, a blanking interval, etc.). Optionally, the implant event message may include a message segment that includes additional/secondary information.

Optionally, the LP 102a, 102b (or other IMD 106) that receives any i2i communication message from another LP 102a, 102b (or other IMD 106) or from an external device 109 may transmit a receive acknowledgement indicating that the receiving LP 102a, 102b (or other IMD 106) received the i2i communication message. In certain embodiments, where an LP (102a, 102b) (or other IMD 106) expects to receive an i2i communication message within a window, and fails to receive the i2i communication message within the window, the LP (102a, 102b) (or other IMD 106) may transmit a failure-to-receive acknowledgement indicating that the receiving LP (102a, 102b) (or other IMD 106) failed to receive the i2i communication message. In other words, an LP 102a, 102b (or other IMD 106) can receive a message from another LP 102a, 102b (or other IMD 106) that includes an indicator (e.g., an error code) in its payload, or header, that indicates to the LP 102a, 102b (or other IMD 106) that the other LP 102a, 102b (or other IMD 106) failed to receive an expected message from the LP 102a, 102b (or other IMD 106). Other variations are also possible and within the scope of the embodiments described herein.

The event messages enable the LPs 102a, 102b to deliver synchronized therapy and additional supportive features (e.g., measurements, etc.). To maintain synchronous therapy, each of the LPs 102a, 102b is made aware (through the event messages) when an event occurs in the chamber containing the other LP 102a, 102b. Some embodiments described herein provide efficient and reliable processes to maintain synchronization between LPs 102a, 102b without maintaining continuous communication between LPs 102a, 102b. In accordance with certain embodiments herein, low power event messages/signaling may be maintained between LPs 102a, 102b synchronously or asynchronously.

For synchronous event signaling, LPs 102a, 102b may maintain synchronization and regularly communicate at a specific interval. Synchronous event signaling allows the transmitter and receivers in each LP 102a,102b to use limited (or minimal) power as each LP 102a, 102b is only powered for a small fraction of the time in connection with transmission and reception. For example, LP 102a, 102b may transmit/receive (Tx/Rx) communication messages in time slots having duration of 10-20 µs, where the Tx/Rx time slots occur periodically (e.g., every 10-20 ms).

LPs 102a, 102b may lose synchronization, even in a synchronous event signaling scheme. As explained herein, features may be included in LPs 102a, 102b to maintain device synchronization, and when synchronization is lost, LPs 102a, 102b undergo operations to recover synchronization. Also, synchronous event messages/signaling may introduce a delay between transmissions which causes a reaction lag at the receiving LP 102a, 102b. Accordingly, features may be implemented to account for the reaction lag.

During asynchronous event signaling, LPs 102a, 102b do not maintain communication synchronization. During asynchronous event signaling, one or more of receivers 120, 122 of LPs 102a, 102b may be "always on" (always awake) to search for incoming transmissions. However, maintaining LP receivers 120, 122 in an "always on" (always awake) state presents challenges as the received signal level often is low due to high channel attenuation caused by the patient's anatomy. Further, maintaining the receivers 120, 122 awake will deplete the battery 114 more quickly than may be desirable.

The asynchronous event signaling methods avoid risks associated with losing synchronization between devices. However, the asynchronous event signaling methods utilize additional receiver current between transmissions. For purposes of illustration only, a non-limiting example is described hereafter. For example, the channel attenuation may be estimated to have a gain of 1/500 to 1/10000. A gain factor may be 1/1000th. Transmit current is a design factor in addition to receiver current. As an example, the system may allocate one-half of the implant communication current budget to the transmitter (e.g., 0.5 µA for each transmitter). When LP 102a, 102b maintains a transmitter in a continuous on-state and the electrode load is 500 ohms, a transmitted voltage may be 2.5 V. When an event signal is transmitted at 2.5 V, the event signal is attenuated as it propagates and would appear at LP 102a, 102b receiver as an amplitude of approximately 0.25 mV.

To overcome the foregoing receive power limit, a pulsed transmission scheme may be utilized in which communication transmissions occur correlated with an event. By way of example, the pulsed transmission scheme may be simplified such that each transmission constitutes a single pulse of a select amplitude and width.

In accordance with certain embodiments herein, LPs 102a, 102b may utilize multi-stage receivers 120, 122 that implement a staged receiver wakeup scheme in order to improve reliability yet remain power efficient. Each of LPs 102a, 102b may include first and second receivers 120 and 122 that operate with different first and second activation protocols and different first and second receive channels. For example, first receiver 120 may be assigned a first activation protocol that is "always on" (also referred to as always awake) and that listens over a first receive channel that has a lower fundamental frequency range/pulse duration (e.g., 1 kHz to 100 kHz / 10 µs to approximately 1 ms) as compared to the fundamental frequency range (e.g., greater than 100 kHz / less than 10 µs per pulse) assigned to the second receive channel.

In accordance with certain embodiments, the first receiver 120 may maintain the first channel active (awake) at all times (including when the second channel is inactive (asleep)) in order to listen for messages from a remote LP 102a, 102b. The second receiver 122 may be assigned a second activation protocol that is a triggered protocol, in which the second receiver 122 becomes active (awake) in response to detection of trigger events over the first receive channel (e.g., when the incoming signal corresponds to the LP wakeup notice, activating the second channel at the local LP 102a, 102b). The terms active, turned on, awake and enabled are used interchangeably herein.

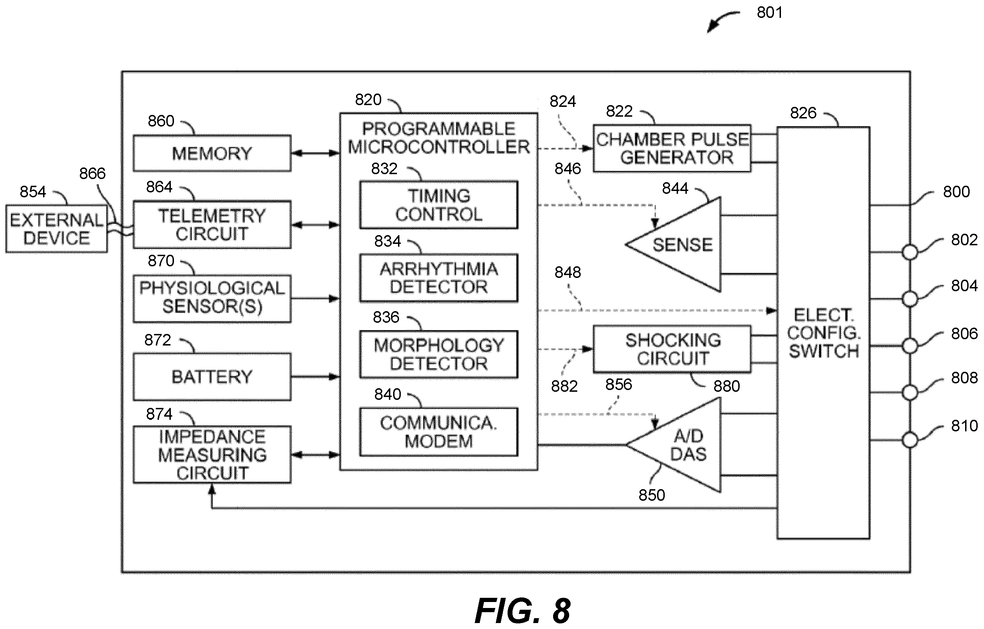

Still referring to FIG. 2, each LP 102a, 102b is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrio-ventricular (AV) delay, atrial interconduction (A-A) delay, or ventricular interconduction (V-V) delay, etc.). Such timing control circuitry may also be used for the timing of refractory periods, blanking intervals, noise detection windows, evoked response windows, alert intervals, marker channel timing, and so on. The controller 112 can further include other dedicated circuitry and/or firmware/software components that assist in monitoring various conditions of the patient's heart and managing pacing therapies. For example, the controller 112 can include an arrhythmia detector, which can be similar to the arrhythmia detector 834 discussed below with reference to FIG. 8.

The controller 112 and the pulse generator 116 may be configured to transmit event messages, via the electrodes 108, in a manner that does not inadvertently capture the heart in the chamber where LP 102a, 102b is located, such as when the associated chamber is not in a refractory state. In addition, an LP 102a, 102b that receives an event message may enter an "event refractory" state (or event blanking state) following receipt of the event message. The event refractory/blanking state may be set to extend for a determined period of time after receipt of an event message in order to avoid the receiving LP 102a, 102b from inadvertently sensing another signal as an event message that might otherwise cause retriggering. For example, the receiving LP 102a, 102b may detect a measurement pulse from another LP 102a, 102b or the programmer 109.

Since the conductive communication receivers 120, 122 (or alternatively, just a single conductive communication receiver) and the pulse generator 116 are used to perform conductive communication, these components can be considered parts of a conductive communication transceiver 124. The conductive communication transceiver 124 can alternatively include alternative and/or additional components that enable an LP 102a, 102b, or other type of IMD 106, to transmit and receive conductive communication messages with another LP 102a, 102b, or other type of IMD 106 and/or with an external device, such as the programmer 109. For an example, while the same pulse generator 116 can be used to produce pacing pulses (for use in pacing a patient's heart) and conductive communication pulses (for use in communicating with another LP 102a, 102b, or other type of IMD 106 or an external device 109), it would also be possible that the conductive communication transceiver 124 can include its own dedicated pulse generator. For another example, as noted above, it would also be possible for the conductive communication transceiver 124 to include only a single receiver, rather than both receivers 120, 122. Other variations are also possible and within the scope of the embodiments described herein.

In accordance with certain embodiments herein, the programmer 109 may communicate over a programmer-to-LP channel, with LP 102a, 102b utilizing the same communication scheme. The external programmer 109 may listen to the event message transmitted between LP 102a, 102b and synchronize programmer to implant communication such that programmer 109 does not transmit communication messages 113 until after an i2i messaging sequence is completed.

In some embodiments, each individual LP 102 can comprise a hermetic housing 110 configured for placement on or attachment to the inside or outside of a cardiac chamber and at least two leadless electrodes 108a, 108b proximal to the housing 110 and configured for bidirectional communication with at least one other device (e.g., an NV-ICD 106) within or outside the body. The electrodes 108a, 108b can be referred to individually as an electrode 108, or collectively as the electrodes 108.

FIG. 2 depicts a single LP 102 (e.g., the LP 102a or 102b) and shows the LP's functional elements substantially enclosed in the hermetic housing 110. The LP 102 has at least two electrodes 108 located within, on, or near the housing 110, for delivering pacing pulses to and sensing electrical activity from the muscle of the cardiac chamber, and for bidirectional communication with at least one other device within or outside the body. Hermetic feedthroughs 130, 131 conduct electrode signals through the housing 110. The housing 110 contains a primary battery 114 to supply power for pacing, sensing, and communication. The housing 110 also contains circuits 132 for sensing cardiac activity from the electrodes 108, receivers 120, 122 for receiving information from at least one other device via the electrodes 108, and the pulse generator 116 for generating pacing pulses for delivery via the electrodes 108 and also for transmitting information to at least one other device via the electrodes 108. The housing 110 can further contain circuits for monitoring device health, for example an optional battery current monitor 136 and an optional battery voltage monitor 138, and can contain circuits for controlling operations in a predetermined manner.

The electrodes 108 can be configured to communicate bidirectionally among the multiple leadless cardiac pacemakers 102 and/or the implanted ICD 106 to coordinate pacing pulse delivery and optionally other therapeutic or diagnostic features using messages that identify an event at an individual pacemaker originating the message and a pacemaker receiving the message react as directed by the message depending on the origin of the message. An LP 102a, 102b that receives the event message reacts as directed by the event message depending on the message origin or location. In some embodiments or conditions, the two or more leadless electrodes 108 can be configured to communicate bidirectionally among the one or more leadless cardiac pacemakers 102 and/or the ICD 106 and transmit data including designated codes for events detected or created by an individual pacemaker. Individual pacemakers can be configured to issue a unique code corresponding to an event type and a location of the sending pacemaker.

In some embodiments, an individual LP 102a, 102b can be configured to deliver a pacing pulse with an event message encoded therein, with a code assigned according to pacemaker location and configured to transmit a message to one or more other leadless cardiac pacemakers via the event message coded pacing pulse. The pacemaker or pacemakers receiving the message are adapted to respond to the message in a predetermined manner depending on type and location of the event.

Moreover, information communicated on the incoming channel can also include an event message from another leadless cardiac pacemaker signifying that the other leadless cardiac pacemaker has sensed a heartbeat or has delivered a pacing pulse, and identifies the location of the other pacemaker. For example, LP 102b may receive and relay an event message from LP 102a to the programmer 109. Similarly, information communicated on the outgoing channel can also include a message to another leadless cardiac pacemaker or pacemakers, or to the ICD 106, that the sending leadless cardiac pacemaker has sensed a heartbeat or has delivered a pacing pulse at the location of the sending pacemaker.

Referring again to FIGS. 1 and 2, the cardiac pacing system 100 may comprise an ICD 106 in addition to one or more LPs 102a, 102b configured for implantation in electrical contact with a cardiac chamber and for performing cardiac rhythm management functions in combination with the implantable ICD 106. The implantable ICD 106 and the one or more LPs 102a, 102b configured for leadless intercommunication by information conduction through body tissue and/or wireless transmission between transmitters and receivers in accordance with the embodiments discussed herein.

In a further embodiment, a cardiac pacing system 100 comprises at least one LP 102a, 102b configured for implantation in electrical contact with a cardiac chamber and configured to perform cardiac pacing functions in combination with the co-implanted ICD 106. Each LP 102 comprise at least two leadless electrodes 108 configured for delivering cardiac pacing pulses, sensing evoked and/or natural cardiac electrical signals, and transmitting information to the co-implanted ICD 106.

As shown in the illustrative embodiments, an LP 102a, 102b can comprise two or more leadless electrodes 108 configured for delivering cardiac pacing pulses, sensing evoked and/or natural cardiac electrical signals, and bidirectionally communicating with the co-implanted ICD 106.

Each LP 102a, 102b can be configured for operation in a respective particular location and to have a respective particular functionality at manufacture and/or by programming by an external programmer. Bidirectional communication among the multiple leadless cardiac pacemakers can be arranged to communicate notification of a sensed heartbeat or delivered pacing pulse event and encoding type and location of the event to another implanted pacemaker or pacemakers. The LP 102a, 102b receiving the communication decode the information and respond depending on location of the receiving pacemaker and predetermined system functionality.

In some embodiments, the LPs 102a, 102b are configured to be implantable in any chamber of the heart, namely either atrium (RA, LA) or either ventricle (RV, LV). Furthermore, for dual-chamber configurations, multiple LPs 102a, 102b may be co-implanted (e.g., one in the RA and one in the RV, one in the RV and one in the coronary sinus proximate the LV). Certain pacemaker parameters and functions depend on (or assume) knowledge of the chamber in which the pacemaker is implanted (and thus with which the LP 102a, 102b is interacting; e.g., pacing and/or sensing). Some non-limiting examples include sensing sensitivity, an evoked response algorithm, use of AF suppression in a local chamber, blanking & refractory periods, etc. Accordingly, each LP 102a, 102b needs to know an identity of the chamber in (or on) which the LP 102a, 102b is implanted, and processes may be implemented to automatically identify a local chamber associated with each LP 102a, 102b.

Processes for chamber identification may also be applied to subcutaneous pacemakers, ICDs 106, with leads and the like. A device with one or more implanted leads, identification and/or confirmation of the chamber into which the lead was implanted could be useful in several pertinent scenarios. For example, for a DR or CRT device, automatic identification and confirmation could mitigate against the possibility of the clinician inadvertently placing the V lead into the A port of the implantable medical device, and vice-versa. As another example, for an SR device, automatic identification of implanted chamber could enable the device and/or programmer to select and present the proper subset of pacing modes (e.g., AAI or VVI), and for the IPG to utilize the proper set of settings and algorithms (e.g., V-AutoCapture vs ACap-Confirm, sensing sensitivities, etc.).

Also shown in FIG. 2, the primary battery 114 has positive terminal 140 and negative terminal 142. Current from the positive terminal 140 of primary battery 114 flows through an optional shunt 144 to an optional regulator circuit 146 to create a positive voltage supply 148 suitable for powering the remaining circuitry of the LP 102. The shunt 144 enables the optional battery current monitor 136 to provide the controller 112 with an indication of battery current drain and indirectly of device health. The illustrative power supply can be a primary battery 114.

Still referring to FIG. 2, the LP 102 is shown as including an optional temperature sensor 152. The temperature sensor can be any one of various different types of well-known temperature sensors, or can be a future developed temperature sensor. For one example, the temperature sensor 152 can be a thermistor, a thermocouple, a resistance thermometer, or a silicon bandgap temperature sensor, but is not limited thereto. Regardless of how the temperature sensor 152 is implemented, it is preferably that the temperature sensed by the sensor is provided to the controller 112 as a digital signal indicative of the blood temperature of the patient within which the LP is implanted. The temperature sensor 152 can be hermetically sealed within the housing 110, but that need not be the case. The temperature sensor 152 can be used in various manners. For example, the temperature sensor 152 can be used to detect an activity level of the patient to adjust a pacing rate, i.e., for use in rate responsive pacing. When a person starts to exercise their core body temperature initially dips, and then after exercising for a prolonged period of time the person's core body temperature will eventually rise. Thereafter, when the person stops exercising their core body temperature will return to its baseline. Accordingly, the controller 112 can be configured to detect an activity level of a patient based on core blood temperature measurements obtained using the temperature sensor 152.

Referring to FIG. 2, the LP 102 is also shown as including an optional accelerometer 154 which can be hermetically contained within the housing 110. The accelerometer 154 can be any one of various different types of well-known accelerometers, or can be a future developed accelerometer. For one example, the accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. For example, the accelerometer 154 can be used to detect an activity level of the patient to adjust a pacing rate, i.e., for use in rate responsive pacing. It would also be possible to use outputs of both the accelerometer 154 and the temperature sensor 152 to monitor the activity level of a patient. Alternatively, or additionally, a patient's activity level can be monitored based on their heart rate, as detected from an IEGM sensed using the electrodes 108, and/or sensed using a plethysmography signal obtained using a plethysmography sensor (not shown) or a heart sound sensor (not shown), but not limited thereto. One or more signals produced and output by the accelerometer 154 may be analyzed with respect to frequency content, energy, duration, amplitude and/or other characteristics. Such signals may or may not be amplified and/or filtered prior to being analyzed. For example, filtering may be performed using lowpass, highpass and/or bandpass filters. The signals output by the accelerometer 154 can be analog signals, which can be analyzed in the analog domain, or can be converted to digital signals (by an analog-to-digital converter) and analyzed in the digital domain. Alternatively, the signals output by the accelerometer 154 can already be in the digital domain. The one or more signals output by the accelerometer 154 can be analyzed by the controller 112 and/or other circuitry. In certain embodiments, the accelerometer 154 is packaged along with an integrated circuit (IC) that is designed to analyze the signal(s) it generates. In such embodiments, one or more outputs of the packaged sensor/IC can be an indication of acceleration along one or more axes. In other embodiments, the accelerometer 154 can be packaged along with an IC that performs signal conditioning (e.g., amplification and/or filtering), performs analog-to-digital conversions, and stores digital data (indicative of the sensor output) in memory (e.g., RAM, which may or may not be within the same package). In such embodiments, the controller 112 or other circuitry can read the digital data from the memory and analyze the digital data. Other variations are also possible, and within the scope of embodiments of the present technology. In accordance with certain embodiments of the present technology, described in additional detail below, a sensor signal produced by the accelerometer 154 of an LP implanted in or on a cardiac chamber can be used to detect mechanical cardiac activity associated with another cardiac chamber.

In various embodiments, LP 102a, 102b can manage power consumption to draw limited power from the battery, thereby reducing device volume. Each circuit in the LP can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery 114.

In some embodiments, the controller 112 in an LP 102 can access signals on the electrodes 108 and can examine output pulse duration from another LP 102 for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

FIG. 3 shows an example form factor of the LPs 102a, 102b. Each LP 102a, 102b can include a hermetic housing 202 (e.g., 110 in FIG. 2) with electrodes 108a and 108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. As noted above, an antenna (e.g., 118) can be at least partially implanted by or as part of the fixation mechanism. The electrodes 108a and 108b are examples of the electrodes 108 shown in and discussed above with reference to FIG. 2.

The housing 202 can also include an electronics compartment 210 within the housing that contains the electronic components necessary for operation of the LP 102a, 102b, including, e.g., a pulse generator, transceiver, a battery, and a processor for operation. The hermetic housing 202 can be adapted to be implanted on or in a human heart, and can be cylindrically shaped, rectangular, spherical, or any other appropriate shapes, for example.

The housing 202 can comprise a conductive, biocompatible, inert, and anodically safe material such as titanium, 316L stainless steel, or other similar materials. The housing 202 can further comprise an insulator 208 disposed on the conductive material to separate electrodes 108a and 108b. The insulator 208 can be an insulative coating on a portion of the housing 202 between the electrodes 108a, 108b, and can comprise materials such as silicone, polyurethane, parylene, or another biocompatible electrical insulator commonly used for implantable medical devices. In the embodiment of FIG. 2, a single insulator 208 is disposed along the portion of the housing 202 between electrodes 108a, 108b. In some embodiments, the housing 202 itself can comprise an insulator instead of a conductor, such as an alumina ceramic or other similar materials, and the electrodes 108a, 108b can be disposed upon the housing 202.

As shown in FIG. 3, the LP 102a, 102b can further include a header assembly 212 to isolate the electrodes 108a, 108b. The header assembly 212 can be made from PEEK, tecothane or another biocompatible plastic, and can contain a ceramic to metal feedthrough, a glass to metal feedthrough, or other appropriate feedthrough insulator as known in the art.

The electrodes 108a, 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 3, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device 205 (and also the electrode 108a in FIG. 3) into contact with stimulable tissue. Electrode 108b can serve as an indifferent electrode for sensing and pacing. The fixation mechanism may be coated partially or in full for electrical insulation, and a steroid-eluting matrix may be included on or near the device to minimize fibrotic reaction, as is known in conventional pacing electrode-leads.

### Implant-to-Implant Event Messaging

LPs 102a, 102b can utilize implant-to-implant (i2i) communication through event messages to coordinate operation with one another in various manners. The terms i2i event messages, and i2i event markers are sometimes used interchangeably herein to refer to event related messages and IMD/IMD, e.g., LP/LP, operation related messages transmitted from an implanted device and directed to another implanted device (although external devices, e.g., a programmer, may also receive i2i event messages). In certain embodiments, LP 102a and LP 102b operate as two independent leadless pacers maintaining beat-to-beat dual-chamber functionality via a "Master/Slave" operational configuration. For descriptive purposes, the ventricular LP 102b shall often be referred to as "vLP" and the atrial LP 102a shall often be referred to as "aLP". The LP 102 that is designated as the master device (e.g., vLP 102b) may implement all or most dual-chamber diagnostic and therapy determination algorithms. For purposes of the following illustration, it is assumed that the vLP 102b is a "master" device, while the aLP 102a is a "slave" device. Alternatively, the aLP 102a may be designated as the master device, while the vLP 102b may be designated as the slave device. The master device orchestrates most or all decision-making and timing determinations (including, for example, rate-response changes).

In accordance with certain embodiments, methods are provided for coordinating operation between first and second leadless pacemakers (LPs 102) configured to be implanted entirely within first and second chambers of the heart. A method transmits an event marker through conductive communication through electrodes 108 located along a housing 110 of the first LP 102, the event marker indicative of one of a local paced or sensed event. The method detects, over a sensing channel, the event marker at the second LP 102. The method identifies the event marker at the second LP 102 based on a predetermined pattern configured to indicate that an event of interest has occurred in a remote chamber. In response to the identifying operation, the method initiates a related action in the second LP 102. In certain embodiments, event messages and/or other types of i2i messages can be alternatively, or additionally, transmitted using RF communication, as will be described in more detail below.

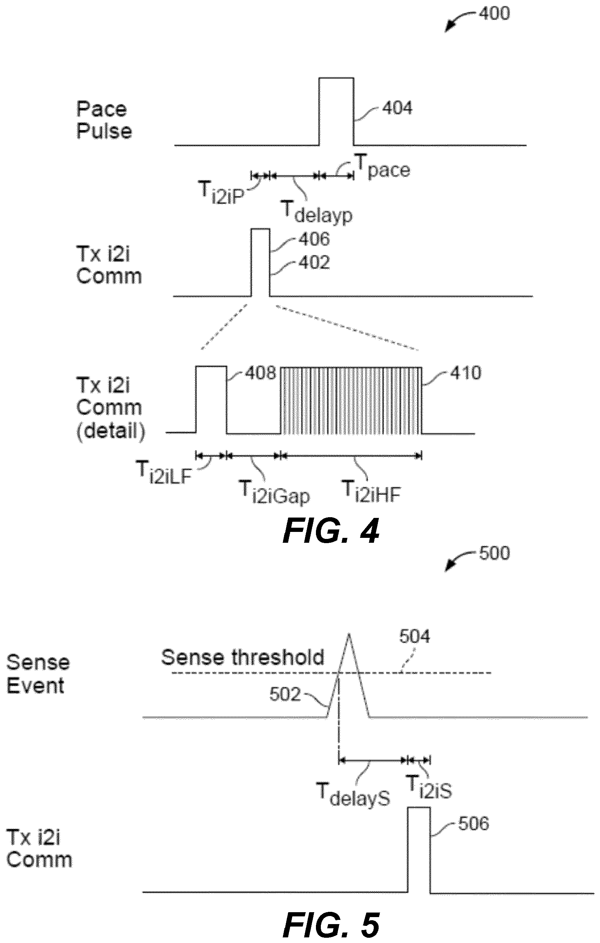

FIG. 4 is a timing diagram 400 demonstrating one example of an i2i conductive communication for a paced event. The i2i conductive communication may be transmitted, for example, from LP 102a to LP 102b, or vice versa. As shown in FIG. 4, in this embodiment, an i2i transmission 402 is sent prior to delivery of a pace pulse 404 by the transmitting LP (e.g., LP 102a). This enables the receiving LP (e.g., LP 102b) to prepare for the remote delivery of the pace pulse. The i2i transmission 402 includes an envelope 406 that may include one or more individual pulses. For example, in this embodiment, envelope 406 includes a low frequency pulse 408 followed by a high frequency pulse train 410. Low frequency pulse 408 lasts for a period T_{i2iLF}, and high frequency pulse train 410 lasts for a period T_{i2iHF}. The end of the low frequency pulse 408 and the beginning of the high frequency pulse train 410 are separated by a gap period, T_{i2iGap}. In alternative embodiments, rather than transmitting the envelope 406 prior to the pacing pulse 404, the envelope 406 can be transmitted during a refractory period that follows the delivery of the pacing pulse.

As shown in FIG. 4, the i2i transmission 402 lasts for a period Ti2iP, and pace pulse 404 lasts for a period T_{pace}. The end of i2i transmission 402 and the beginning of pace pulse 404 are separated by a delay period, T_{delayP}. The delay period may be, for example, between approximately 0.0 and 10.0 milliseconds (ms), particularly between approximately 0.1 ms and 2.0 ms, and more particularly approximately 1.0 ms. The term approximately, as used herein, means +/- 10% of a specified value.

FIG. 5 is a timing diagram 500 demonstrating one example of an i2i communication for a sensed event. The i2i communication may be transmitted, for example, from LP 102a to LP 102b. As shown in FIG. 5, in this embodiment, the transmitting LP (e.g., LP 102a) detects the sensed event when a sensed intrinsic activation 502 crosses a sense threshold 504. A predetermined delay period, T_{delayS}, after the detection, the transmitting LP transmits an i2i transmission 506 that lasts a predetermined period T_{i2iS}. The delay period may be, for example, between approximately 0.0 and 10.0 milliseconds (ms), particularly between approximately 0.1 ms and 2.0 ms, and more particularly approximately 1.0 ms.

As with i2i transmission 402, i2i transmission 506 may include an envelope that may include one or more individual pulses. For example, similar to envelope 406, the envelope of i2i transmission 506 may include a low frequency pulse followed by a high frequency pulse train. In certain embodiments, the i2i transmission 506 is transmitted during a refractory period that follows the sensed event.

Optionally, wherein the first LP 102a is located in an atrium and the second LP 102b is located in a ventricle, the first LP 102a produces an AS/AP event marker to indicate that an atrial sensed (AS) event or atrial paced (AP) event has occurred or will occur in the immediate future. For example, the AS and AP event markers may be transmitted following the corresponding AS or AP event. Alternatively, the first LP 102a may transmit the AP event marker slightly prior to delivering an atrial pacing pulse. Alternatively, wherein the first LP 102a is located in an atrium and the second LP 102b is located in a ventricle, the second LP 102b initiates an atrioventricular (AV) interval after receiving an AS or AP event marker from the first LP 102a; and initiates a post atrial ventricular blanking (PAVB) interval after receiving an AP event marker from the first LP 102a.

Optionally, the first and second LPs 102 may operate in a "pure" master/slave relation, where the master LP delivers "command" markers in addition to or in place of "event" markers. A command marker directs the slave LP to perform an action such as to deliver a pacing pulse and the like. For example, when a slave LP 102a is located in an atrium and a master LP 102b is located in a ventricle, in a pure master/slave relation, the slave LP 102a delivers an immediate pacing pulse to the atrium when receiving an AP command marker from the master LP 102b.

In accordance with some embodiments, communication and synchronization between the aLP 102a and vLP 102b is implemented via conducted communication of markers/commands in the event messages (per i2i communication protocol). As explained above, conducted communication represents event messages transmitted from the sensing/pacing electrodes at frequencies outside the RF (e.g., Wi-Fi or BLE) frequency range. The figures and corresponding description below illustrate non-limiting examples of markers that may be transmitted in event messages. The figures and corresponding description below also include the description of the markers and examples of results that occur in the LP that receives the event message. Table 1 represents example event markers sent from the aLP 102a to the vLP 102b, while Table 2 represents example event markers sent from the vLP 102b to the aLP 102a. In the master/slave configuration, AS event markers are sent from the aLP 102a each time that an atrial event is sensed outside of the post ventricular atrial blanking (PVAB) interval or some other alternatively-defined atrial blanking period. The AP event markers are sent from the aLP 102a each time that the aLP 102a delivers a pacing pulse in the atrium. The aLP 102a may restrict transmission of AS markers, whereby the aLP 102a transmits AS event markers when atrial events are sensed both outside of the PVAB interval and outside the post ventricular atrial refractory period (PVARP) or some other alternatively-defined atrial refractory period. Alternatively, the aLP 102a may not restrict transmission of AS event markers based on the PVARP, but instead transmit the AS event marker every time an atrial event is sensed.

**Table 1**

| "A2V" Markers / Commands (i.e., from aLP to vLP) | | |
|---|---|---|
| *Marker* | *Description* | *Result in vLP* |
| AS | Notification of a sensed event in atrium (if not in PVAB or PVARP) | • Initiate AV interval (if not in PVAB or PVARP) |
| AP | Notification of a paced event in atrium | • Initiate PAVB |
| | | • Initiate AV interval (if not in PVARP) |

As shown in Table 1, when an aLP 102a transmits an event message that includes an AS event marker (indicating that the aLP 102a sensed an intrinsic atrial event), the vLP 102b initiates an AV interval timer. If the aLP 102a transmits an AS event marker for all sensed events, then the vLP 102b would preferably first determine that a PVAB or PVARP interval is not active before initiating an AV interval timer. If however the aLP 102a transmits an AS event marker only when an intrinsic signal is sensed outside of a PVAB or PVARP interval, then the vLP 102b could initiate the AV interval timer upon receiving an AS event marker without first checking the PVAB or PVARP status. When the aLP 102a transmits an AP event marker (indicating that the aLP 102a delivered or is about to deliver a pace pulse to the atrium), the vLP 102b initiates a PVAB timer and an AV interval time, provided that a PVARP interval is not active. The vLP 102b may also blank its sense amplifiers to prevent possible crosstalk sensing of the remote pace pulse delivered by the aLP102a.

**Table 2**

| "V2A" Markers / Commands (*i.e.,* from vLP to aLP) | | |
|---|---|---|
| *Marker* | *Description* | *Result in aLP* |
| VS | Notification of a sensed event in ventricle | • Initiate PVARP |
| VP | Notification of a paced event in ventricle | • Initiate PVAB |
| | | • Initiate PVARP |
| AP | Command to deliver immediate pace pulse in atrium | • Deliver immediate pace pulse to atrium |

As shown in Table 2, when the vLP 102b senses a ventricular event, the vLP 102b transmits an event message including a VS event marker, in response to which the aLP 102a may initiate a PVARP interval timer. When the vLP 102b delivers or is about to deliver a pace pulse in the ventricle, the vLP 102b transmits VP event marker. When the aLP 102a receives the VP event marker, the aLP 102a initiates the PVAB interval timer and also the PVARP interval timer. The aLP 102a may also blank its sense amplifiers to prevent possible crosstalk sensing of the remote pace pulse delivered by the vLP 102b. The vLP 102b may also transmit an event message containing an AP command marker to command the aLP 102a to deliver an immediate pacing pulse in the atrium upon receipt of the command without delay.

The foregoing event markers are examples of a subset of markers that may be used to enable the aLP 102a and vLP 102b to maintain full dual chamber functionality. In one embodiment, the vLP 102b may perform all dual-chamber algorithms, while the aLP 102a may perform atrial-based hardware-related functions, such as PVAB, implemented locally within the aLP 102a. In this embodiment, the aLP 102a is effectively treated as a remote 'wireless' atrial pace/sense electrode. In another embodiment, the vLP 102b may perform most but not all dual-chamber algorithms, while the aLP 102a may perform a subset of diagnostic and therapeutic algorithms. In an alternative embodiment, vLP 102b and aLP 102a may equally perform diagnostic and therapeutic algorithms. In certain embodiments, decision responsibilities may be partitioned separately to one of the aLP 102a or vLP 102b. In other embodiments, decision responsibilities may involve joint inputs and responsibilities.

Messages that are transmitted between LPs 102 (e.g., the aLP 102a and the vLP 102b) can be referred to herein generally as i2i messages, since they are implant-to-implant messages. As noted above, such messages can include event markers that enable one LP 102 to inform the other LP 102 of a paced event or a sensed event. For example, in certain embodiments, whenever the aLP 102a senses an atrial event or paces the right atrium, the aLP 102a will transmit an i2i message to the vLP 102b to inform the vLP 102b of the sensed or paced event in the atrium. In response to receiving such an i2i message, the vLP 102b may start one or more timers that enable the vLP 102b to sense or pace in the right ventricle. Similarly, the vLP 102b may transmit an i2i message to the aLP 102a whenever the vLP 102b senses a ventricular event or paces the right ventricle.

The i2i messages that are sent between LPs 102 may be relatively short messages that simply allow a first LP 102 to inform a second LP 102 of an event that was sensed by the first LP 102 or caused (paced) by the first LP 102, and vice versa. Such i2i messages can be referred to herein as event marker i2i messages, or more succinctly as event i2i messages. The i2i messages that are sent between LPs 102, in certain instances, can be extended i2i messages that include (in addition to an event marker) an extension. In certain embodiments, an extended i2i message includes an event marker (e.g., 9 bits), followed by an extension indicator (e.g., 2 bits), followed by an extended message payload portion (e.g., 17 bits), followed by a cyclic redundancy check (CRC) code (e.g., 6 bits) or some other type of error detection code.

In certain embodiments, whenever an i2i message is sent by an LP 102 (or other type of IMD, such as a S-ICD 106), the i2i message will include an extension indicator so that the receiving LP 102 knows whether or not the i2i message it receives includes an extension portion. In such embodiments, even a relatively short event i2i message will include an extension indicator. The extension indicator (e.g., 2 bits) is used by the LP102 (or other IMD 106) sending the i2i message to indicate, to the LP 102 receiving the i2i message, whether or not the i2i message is an extended i2i message. In certain embodiments, if the LP 102 receiving an i2i message determines based on the extension indicator bits that the received i2i message is not an extended i2i message, then the LP 102 receiving the i2i message can ignore any bits that follow the extension bits. In such a case, the LP 102 receiving the i2i message only decodes the event marker. On the other hand, if the LP 102 receiving an i2i message determines based on the extension indicator bits that the received i2i message is an extended i2i message, then the LP 102 receiving the i2i message will also decode the bits that follow the extension bits, and determine based on a CRC code (or other type of error detection code), whether the i2i message is a valid message. If the extended i2i message is a valid i2i message, then the LP 102 receiving the extended i2i message will as appropriate modify its operation, update parameters, and/or the like, based on information included in the extended i2i message. In certain embodiments, event i2i messages that are not extended i2i messages do not include any error detection and correction code.

In an extended i2i message, the event marker bits and the extension indicator bits are located, respectively, in an event marker field and an extension indicator field of an i2i message packet. In certain embodiments, the extended portion (that follows the event marker bits and the extension indicator bits) includes message bits (in a message field) and rate indicator bits (in a rate indicator field), which are parts of the payload. The payload can alternatively, or additionally, include other types of fields, such as an acknowledgement field that is used in certain situations for one LP 102 to acknowledge reception of an i2i message from another LP 102 of certain (e.g., critical) types of messages.

More generally, various different types of information may be included within the payload of an extended i2i message. For one example, the payload can include a pacing rate indicator that enables one LP 102 to inform another LP 102 of a pacing rate. For example, assume that an LP system 100 provides rate responsive pacing, wherein a pacing rate is adjusted in dependence on a patient's physical activity as detected, e.g., using an accelerometer 154, temperature sensor 152, and/or other type of sensor of an LP 102. In such an LP system 100, the vLP 102b may inform the aLP 102a of the rate at which the patient's heart should be paced so that the aLP 102a and vLP 102b can perform synchronized pacing. To achieve this, the vLP 102b can send a pacing rate indicator to the aLP 102a in the payload of an extended i2i message. The pacing rate indicator can, e.g., be a value indicating a pacing rate value (e.g., 80 bpm), a code that the aLP 102a that can look up (e.g., in a stored look up table) and corresponds to a pacing rate value, or a value that the aLP 102a feeds into an equation to determine the pacing rate, but is not limited thereto. Alternatively, the pacing rate indicator can be beat-to-beat interval value (e.g., 0.75 seconds), a code that the aLP 102a can look up and corresponds to a beat-to-beat interval value, or a value that the aLP 102a feeds into an equation to determine the beat-to-beat interval, but is not limited thereto. Other variations are also possible and within the scope of the embodiments described herein.

### Methods for Implementing i2i Noise Reversion with Checking

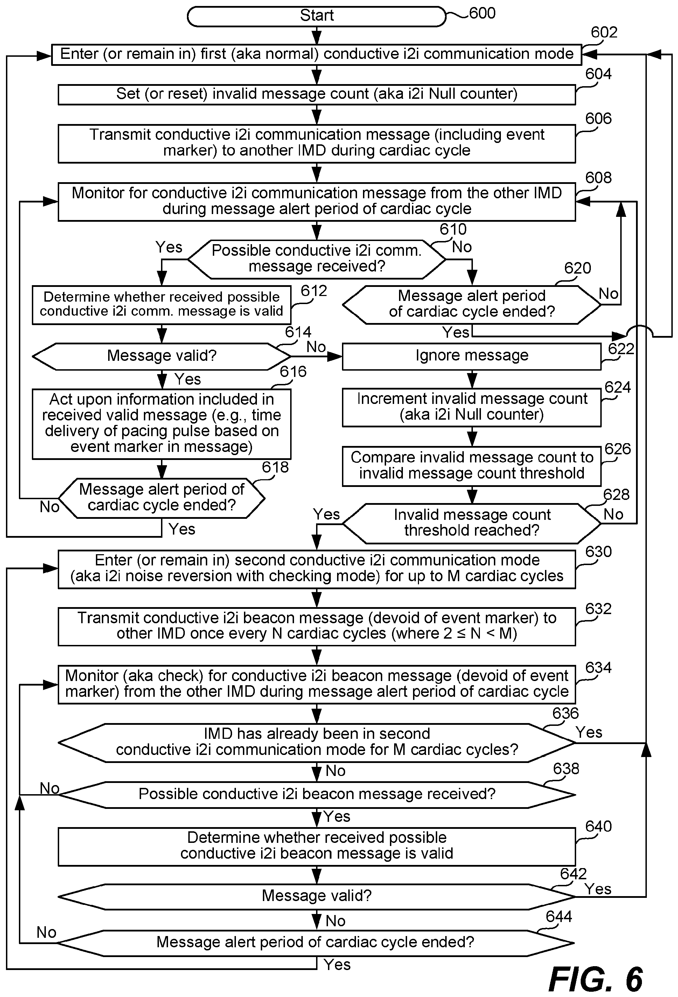

FIG. 6 is a high level flow diagram that is used to summarize methods for improving communication between IMDs (e.g., first and second LPs 102), according to certain embodiments of the present technology. More specifically, the methods summarized with reference to FIG. 6 are for use by an IMD (e.g., a first LP 102) that communicates with another IMD (e.g., a second LP 102) using conductive implant-to-implant (i2i) communications. For a more specific example, the first LP can be an aLP 102a and the second LP can be a vLP 102b, or vice versa. Referring to FIG. 6, block 600, labeled "Start", is the entry point for the method. Step 602 involves the IMD entering (or remaining in) a first conductive i2i communication mode. In certain embodiments, the first conductive i2i communication mode is a normal conductive i2i communication mode, e.g., a default communication mode of the IMD. In accordance with certain embodiments, while an IMD is operating in its first conductive i2i communication mode the IMD will transmit a conductive i2i communication message, which includes an event marker, to the other IMD once per cardiac cycle, so that the other IMD can time its pacing and/or other functions based on the event marker it receives. As was discussed above with reference to the Table 1, where an IMD is an aLP 102a, the event marker can indicate that the aLP 102a sensed an intrinsic event in the atrium (i.e., the event marker can be an AS event marker), or that the aLP 102a caused a paced event in the atrium (i.e., the event marker can be an AP event marker). As was discussed above with reference to the Table 2, where an IMD is a vLP 102b, the event marker can indicate that the vLP 102b sensed an intrinsic event in a ventricle (i.e., the event marker can be a VS event marker), or that the vLP 102b caused a paced event in the ventricle (i.e., the event marker can be a VP event marker). A conductive i2i communication message that includes an AS or VS event marker, or more generally a sensed event marker, can be transmitted a short delay after the sensed event was detected. A conductive i2i communication message that includes an AP or VP event marker, or more generally a paced event marker, can be transmitted a short delay before (or after) the pacing stimulation is delivered. Other variations are also possible and within the scope of the embodiments described herein.

Step 604 involves the IMD setting (or resetting) an invalid message count, which can also be referred to herein as an i2i Null counter. In other words, at a step 604, an invalid message count is set, or reset, to its initial value, which can be zero, but is not limited thereto. Step 606 involves the IMD transmitting a conductive i2i communication message, which includes an event marker, to the other IMD during a cardiac cycle. During the same cardiac cycle, at step 608, the IMD monitors for a conductive i2i communication message (that includes an event marker) from the other IMD during a message alert period of the cardiac cycle. In certain embodiments, the message alert period is related to a cardiac cycle, and may occur during an entire cardiac cycle, or just a portion of a cardiac cycle. For example, where an IMD performing the method is implanted in a ventricle, a message alert period can be a portion of a cardiac cycle that follows an AV delay, a blanking period and/or a relative refractory period, but is not limited thereto. For another example, where an IMD performing the method is implanted in an atrium, a message alert period can be a portion of a cardiac cycle that follows a post ventricular atrial blanking (PVAB) period, a post ventricular atrial refractory period (PVARP) and/or some other alternatively-defined atrial refractory period.

At step 610 there is a determination of whether a possible conductive i2i communication message is received. If the answer to the determination at step 610 is No (i.e., if a possible conductive i2i communication message was not received), then at step 620 there is a determination of whether the message alert period has ended. If the answer to the determination at step 620 is No (i.e., if the message alert period has not ended), then flow returns to step 608. If the answer to the determination at step 620 is Yes (i.e., if the message alert period has ended), then flow returns to step 602. In accordance with certain embodiments, in order to conserver power, and thus a battery and/or device life, during the period of time between when a message alert period has ended and when a next message alert period begins, a receiver and/or transmitter (and/or one or more other components of an IMD) can be disabled or put into a low power mode.

In accordance with certain embodiments, there is a determination that a possible conductive i2i communication message is received at step 610 (i.e., the answer to the determination at step 610 will be Yes), when a magnitude of a signal detected within a passband of the first receiver 120 (e.g., a passband from approximately 1kHz to approximately 100kHz) exceeds a detection threshold. In specific embodiments, the answer to the determination at step 610 will be Yes when an IMD detects a wakeup notice (aka a "wakeup pulse"), or what appears to be a wakeup notice, from the other IMD.

If the answer to the determination at step 610 is Yes (i.e., if a possible conductive i2i communication message was received), then at step 612 there is a determination of whether the received possible conductive i2i communication message is valid. The term "message", as used herein, can refer to an actual sent message that is received and is capable of being decoded by the second receiver 122, an actual sent message that is received but is too noisy to be decoded by the second receiver 122, an actual sent message that is received but due to noise it is decoded mistakenly for a different message, noise that is initially mistaken for being an actual message but is sufficiently different than an actual message so that it cannot be decoded by the second receiver 122, as well as noise that is received and is mistaken for being an actual message and is decoded by the IMD because the noise is sufficiently similar to an actual message. The term "valid message", as used herein, can refer to an actual sent message that is received and is capable of being decoded by the second receiver 122, an actual sent message that is received but due to noise it is decoded mistakenly for a different message (this may occur in rare circumstances), or noise that is received and is mistaken for being an actual message and is decoded by the IMD because the noise is sufficiently similar to an actual message (this may occur in very rare circumstances). The latter two types of a "valid message", which may occur in rare or very rare circumstances, are examples false positives. Accordingly, it is possible that a "valid message" is not an actual message, or is an actual message that has been decoded incorrectly. The term "invalid message", as used herein, can refer to an actual sent message that is received but is too noisy to be decoded by the second receiver 122, as well as noise that is initially mistaken for being an actual message but is sufficiently different than an actual message so that it cannot be decoded by the second receiver 122. In accordance with certain embodiments, the determination of whether a message is valid or invalid can be performed by a controller (e.g., processor) that performs decoding and error detection and/or correction.

In accordance with certain embodiments, step 612 involves performing a message validation operation on the possible conductive i2i communication message that was received. Such a message validation operation can involve decoding the received possible conductive i2i communication message and determining whether the decoded message is one of one or more (e.g., four) possible valid messages. Additionally, in accordance with certain embodiments, each valid communication message includes a cyclic redundancy check (CRC) code (or some other type of error detection code), and the message validation operation also includes using the error detection code (e.g., CRC code) to determine whether the received possible conductive i2i communication message is valid.

At step 614 there is a determination of whether the received message was determined to be valid at step 612. While steps 614 and 612 are shown as separate steps in FIG. 6, these two steps could have instead been shown as a single step.

Still referring to FIG. 6, if the answer to the determination at step 614 is No (i.e., if a valid message was not received), then flow goes to step 622, and the received message (which was determined to have been not valid, or invalid) is ignored. At step 624 the invalid message count (aka i2i Null counter) is incremented. For example, the equation imc = imc + 1 can be used at step 624, where imc is the invalid message count, which is set or reset each time step 604 is performed. At step 626 the invalid message count (imc) is compared to an invalid massage count threshold (imct), and at step 628 there is a determination of whether the invalid message count threshold (imct) has been reached. If the answer to the determination at step 628 is No (i.e., if the invalid message count threshold has not been reached), then flow returns to step 608 and monitoring for another possible message occurs. If the answer to the determination at step 628 is Yes (i.e., if the invalid message count threshold has been reached), that is indicative of electromagnetic interference (EMI) and/or other noise being present, and thus, at step 630 a second conductive i2i communication mode, which can be referred to herein as an i2i Noise Reversion with checking mode, is entered. In other words, there is a switch from the first conductive i2i communication mode to the second conductive i2i communication mode in response to the answer to the determination at step 628 being Yes. While steps 626 and 628 are shown as two separate steps, these two steps could have instead been shown as a single step.

Once the IMD has entered (and thereby switched to) the second conductive i2i communication mode at an instance of step 630, while the IMD is in the second conductive i2i communication mode, at step 632, the IMD transmits an i2i beacon message (which is devoid of an event marker) to the other IMD, preferably once every N cardiac cycles, where N is an integer that is greater than or equal to 2. In accordance with certain embodiments, N is within the range of 2 to 60, but is not limited thereto. In certain such embodiments, N is within the range of 4 to 30, e.g., is 8 or 10, but is not limited thereto. Where the method being described is performed independently by an aLP 102a implanted in an atrium, as well as by a vLP 102b implanted in a ventricle, what the aLP 102a considers a cardiac cycle may differ from what the vLP 102b considers a cardiac cycle, because the aLP 102a and the vLP 102b have different frames of reference. For example, the aLP 102a may consider a cardiac cycle as the period between two atrial (paced or sensed) events, whereas the vLP 102b may consider a cardiac cycle as the period between two ventricular (paced or sensed) events. Further, while the IMD is in the second conductive i2i communication mode, at step 634 the IMD monitors for (and more specifically, attempts to receive) an i2i beacon message (which is devoid of an event marker) from the other IMD during a message alert period of a cardiac cycle. The message alert period of a cardiac cycle was already described above during the discussion of step 608, and thus need not be described again. In accordance with certain embodiments, the second conductive i2i communication mode is the i2i Noise Reversion with checking mode.

A reason an IMD transmits the conductive i2i beacon message to another IMD preferably every N cardiac cycles, and attempts to receive a conductive i2i beacon message from the other IMD, is so that the IMD can determine in an power efficient manner whether the noise (that had caused the IMD to switch from the first conductive i2i communication mode to the second conductive i2i communication mode) is still present, or has subsided to the point that the IMDs should switch back to the first conductive i2i communication mode prior to the IMD being in the second conductive i2i communication mode for M cardiac cycles. In other words, the conductive i2i beacon messages are used to test whether noise has subsided to the extent that an IMD can switch back to its the first conductive i2i communication earlier than waiting the entire M cardiac cycles. This is beneficial, assuming the first conductive i2i communication mode enables a system 100 to operate in a preferred manner compared to how the system 100 operates using the second conductive i2i communication mode. For example, using the first conductive i2i communication mode an aLP 102a and a vLP 102b of a dual-chamber LP system 100 can maintain beat-to-beat AV synchrony, which may not be maintained using the second conductive i2i communication mode during which one or both of the LPs 102a, 102b may perform pacing in accordance with a safe pacing mode that does not rely on i2i communication. Depending on the specific implementation, each IMD (e.g., aLP 102a and vLP 102b) can transmit the same conductive i2i beacon message, or the conductive i2i beacon message transmitted by one IMD (e.g., an aLP 102a) may differ from that transmitted by another IMD (e.g., a vLP 102b). Either implementation will work, so long as the IMD that receives the conductive i2i beacon message from another IMD knows the predetermined value or code of the conductive i2i beacon message.

Assuming the IMDs are first and second LPs 102 (e.g., an aLP 102a and a vLP 102b), when the LPs 102 are able to successfully communicate with one another using conductive i2i communication it is preferred that the LPs 102 are in the first conductive i2i communication mode because the LPs 102 are able to provide coordinated operations (e.g., coordinated dual chamber therapy) when in the LPs 102 are in the first conductive i2i communication mode. By contrast, in accordance with certain embodiments, when the LPs 102 are in the second conductive i2i communication mode they operate in a safe pacing mode that does not depend on valid conductive i2i communication messages being received by the first LP 102 from the second LP 102, and vice versa.

In accordance with certain embodiments, when an IMD is in the second conductive i2i communication mode the IMD consumes on average less power per cardiac cycle than the IMD does when it is in the first conductive i2i communication mode. In certain such embodiments, the second conductive i2i communication mode consumes on average at least fifty percent less power per cardiac cycle than the first conductive i2i communication mode. One reason that the IMD consumes less power in the second conductive i2i communication mode is that the IMD transmits conductive i2i communication messages less frequently when the IMD is in the second conductive i2i communication mode compared to when the IMD is in the first conductive i2i communication mode. Another reason that the IMD consumes less power in the second conductive i2i communication mode is that the conductive i2i beacon message (which is devoid of an event marker) that is sent once every N cardiac cycles when the IMD is in the second conductive i2i communication mode includes less conductive i2i communication pulses than the conductive i2i communication messages (which include an event marker) that is sent when the IMD is in the first conductive i2i communication mode. Further, in accordance with certain embodiments, while an IMD is in the first conductive i2i communication mode, one or more of the outgoing conductive i2i communication messages transmitted by the IMD includes a payload, and by contrast, while the IMD is in the second conductive i2i communication mode the outgoing conductive i2i beacon messages transmitted by the IMD are devoid of a payload.

Accordingly, a reason that an IMD switches from the first conductive i2i communication mode to the second conductive i2i communication mode is so that in the presence of excessive noise that adversely affects conductive i2i communication between IMDs, the IMDs can save power by attempting to communicate less frequently with shorter messages. In other words, if IMDs are having difficulty communicating with one another for a duration of time due to excessive noise, it is beneficial for the IMDs to not waste power attempting to communicate with one another, but rather, to reduce their power consumption for at least a duration of time. Additionally, in accordance with certain embodiments, during those N-1 cardiac cycles that an IMD does not transmit a conductive i2i beacon message to the other IMD, the IMD can disable its transmitter to further conserve power.

Still referring to FIG. 6, at step 636 an IMD determines whether it has already been in the second conductive i2i communication mode for M cardiac cycles, where M is greater than N. In accordance with certain embodiments, M is preferably at least 4 times greater than N, and is more preferably at least 10 times greater than N, but is not limited thereto. In accordance with certain embodiments, M is within the range of 100 to 300, e.g., M = 255. If the answer to the determination at step 636 is No, then flow goes to step 638. If the answer to the determination at step 636 is Yes, then flow returns to step 602 and then IMD returns (at least temporarily) to the first conductive i2i communication mode.

As noted above, if the answer to the determination at step 636 is No, then flow goes to step 638. At step 638 there is a determination of whether a possible conductive i2i beacon message has been received. While steps 634 and 638 are shown as separate steps in FIG. 6, these two steps could have instead been shown as a single step that occurs prior to or after step 636.

If the answer to the determination at step 638 is No, then flow returns to step 634, as shown in FIG. 6. If the answer to the determination at step 638 is Yes, then flow goes to step 640, and the IMD determines whether the received possible conductive i2i beacon message is valid. In accordance with certain embodiments, there is a determination that a possible conductive i2i beacon message is received at step 638 (i.e., the answer to the determination at step 638 will be Yes), when a magnitude of a signal detected within a passband of the first receiver 120 (e.g., a passband from approximately 1 kHz to approximately 100 kHz) exceeds a detection threshold. In specific embodiments, the answer to the determination at step 638 will be Yes when an IMD detects a wakeup notice (aka a "wakeup pulse"), or what appears to be a wakeup notice, from the other IMD.

In accordance with certain embodiments, step 640 involves performing a message validation operation on the possible conductive i2i beacon message that was received. Such a message validation operation can involve decoding the received possible conductive i2i beacon message and determining whether the decoded message is indeed the known beacon message. Additionally, in accordance with certain embodiments, each valid beacon message includes a CRC code (or some other type of error detection code), and the message validation operation performed at step 640 also includes using the error detection code (e.g., CRC code) to determine whether the received possible conductive i2i beacon message is valid.

At step 642 there is a determination of whether the received possible conductive i2i beacon message is valid. If the answer to the determination at step 642 is No, then flow goes to step 644. If the answer to the determination at step 642 is Yes, then flow returns to step 602, at which the IMD switches back to the first conductive i2i communication mode. This switch back to the first conductive i2i communication mode occurs because it is assumed that the noise level must have sufficiently subsided if the IMD was able to successfully receive a valid conductive i2i beacon message. While steps 640 and 642 are shown as separate steps in FIG. 6, these two steps could have instead been shown as a single step. At step 644 there is a determination of whether the message alert period has ended. If the answer to the determination at step 644 is No, then flow returns to step 634. If the answer to the determination at step 644 is Yes, then flow returns to step 630. The determination that occurs at step 644 is the same or similar to the determination that occurs at step 620, and thus, details of step 644 can be appreciated from the above discussion of step 620.

Returning to the discussion of step 614 in FIG. 6, if the answer to the determination at step 614 is Yes (i.e., if a valid message was received), then flow goes to step 616, and information included in the valid message is acted upon. Where the IMD (e.g., LP 102b) performing the method is implanted in a ventricle, the information included in the valid message (e.g., received from another IMD, e.g., LP 102a, implanted in an atrium) can be an atrial sense (AS) notification of a sensed event in the atrium that causes the IMD to initiate an AV interval, or an atrial pace (AP) notification of a paced event in atrium that causes the IMD to initiate a post ventricular atrial blanking (PVAB) interval, some other alternatively-defined atrial blanking period, or an AV interval (if not in PVARP). Where the IMD (e.g., aLP 102a) performing the method is implanted in an atrium, the information included in the valid message (e.g., received from another IMD, e.g., vLP 102b, implanted in a ventricle) can be a ventricular sense (VS) notification of a sensed event in a ventricle that causes the IMD to initiate a PVARP interval, or an ventricular pace (VP) notification of a paced event in ventricle that causes the IMD to initiate a PVAB interval or a PVARP interval, or an atrial pace (AP) command that causes the IMD to immediately deliver a pace pulse in the atrium. These are just a few examples, which are not intended to be all encompassing.

Still referring to FIG. 6, after (or while) step 616 is being performed, at step 618 there is a determination of whether the message alert period has ended. If the answer to the determination at step 620 is No (i.e., if the message alert period has not ended), then flow returns to step 608. If the answer to the determination at step 620 is Yes (i.e., if the message alert period has ended), then flow returns to step 602. As can be appreciated from FIG. 6, steps 618 and 620 are the same, and thus, the flow diagram in FIG. 6 can be redrawn to include just one of those steps. In certain embodiments it is possible that more than one valid message can be received during a same message alert period. For example, if a vLP 102b implanted within a ventricle detects an R wave and soon thereafter detects a premature ventricular contraction (PVC), this may results in the vLP 102b transmitting more than one valid message during the same cardiac cycle, and this may result in an aLP 102a implanted within an atrium receiving more than one valid message within a same message alert period.

The invalid message count threshold (imct), referred to at steps 626 and 628, can be a value that is either set by default or by a physician or clinician, which once set, is not changed on the fly by the IMD that is performing the method summarized with reference to FIG. 6. In alternative embodiments, the IMD can change the invalid message count threshold (imct) on the fly, in dependence on whether a valid message was detected during an immediately preceding message alert period. More specifically, the invalid message count threshold (imct) can be reduced if a valid message was not detected during an immediately preceding message alert period, compared to if a valid message was detected during the immediately preceding message alert period. Accordingly, this method variant effectively makes it more difficult for an IMD in the second conductive i2i communication mode to return to the first conductive i2i communication mode, assuming the IMD is configured to remain in the second conductive i2i communication mode (once entered) until a valid conductive i2i beacon message (or a specified number of valid conductive i2i beacon messages) are thereafter received. Alternatively, the invalid message count threshold (imct) can be increased if a valid message was not detected during an immediately preceding message alert period, compared to if a valid message was detected during the immediately preceding message alert period. More generally, in certain embodiments the invalid message count threshold can be specified in dependence on whether a valid message was detected during an immediately preceding message alert period.

If an IMD implements the flow diagram of FIG. 6, the IMD will switch from the second conductive i2i communication mode back to the first conductive i2i communication mode (prior to the IMD being in the second conductive i2i communication mode for M cardiac cycles), if the IMD detect a valid incoming conductive i2i beacon message prior to the IMD being in the second conductive i2i communication mode for M cardiac cycles. In other words, if the answer to the determination at step 642 is Yes prior to the answer to the determination at step 636 being Yes, then IMD will switch from the second conductive i2i communication mode back to the first conductive i2i communication mode, prior to the IMD being in the second conductive i2i communication mode for M cardiac cycles. In alternative embodiments, an IMD must detect a valid incoming conductive i2i beacon message in at least a specified number X of cardiac cycles prior to the IMD being in the second conductive i2i communication mode for M cardiac cycles, in order for the IMD to switch from the second conductive i2i communication mode back to the first conductive i2i communication mode (prior to the IMD being in the second conductive i2i communication mode for M cardiac cycles), wherein X is a specified integer that is equal to or greater than 2. In certain such alternative embodiments, the IMD must detect a valid incoming conductive i2i beacon message in at least a specified number X out of Y (e.g., 2 out of 4) cardiac cycles prior to the IMD being in the second conductive i2i communication mode for M cardiac cycles, in order for the IMD to switch from the second conductive i2i communication mode back to the first conductive i2i communication mode (prior to the IMD being in the second conductive i2i communication mode for M cardiac cycles), wherein X is a specified integer that is equal to or greater than 2, and Y is a specified integer that is greater than X. Other variations are also possible and within the scope of the embodiments described herein. For example, in certain embodiments an IMD must detect a specified number of valid incoming conductive i2i beacon messages within a specified duration of time prior to the IMD being in the second conductive i2i communication mode for M cardiac cycles (or a further specified duration of time), in order for the IMD to switch from the second conductive i2i communication mode back to the first conductive i2i communication mode (prior to the IMD being in the second conductive i2i communication mode for M cardiac cycles or the further specified duration of time).

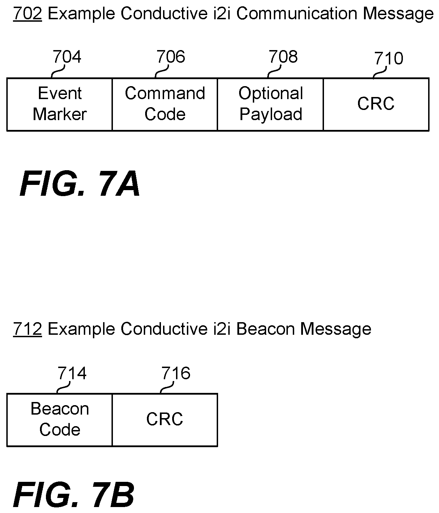

FIG. 7A illustrates example contents of a conductive i2i communication message 702, and FIG. 7B illustrates example contents of a conductive i2i beacon message 712, according to certain embodiments of the present technology. Referring to FIG. 7A, the example conductive i2i communication message 702 is shown as including an event marker 704, a command code 706, an optional payload 708, and a CRC 714 (or other type of error detection code). The example conductive i2i beacon message 712 is shown as including a beacon code 714 and a CRC 716. Another type of error detection code (e.g., a parity code, a checksum, or a repetition code, but not limited thereto) can be used in place of the CRC 710, 716, so long as the error detection code can be used to determine the validity of received messages. As can be appreciated from FIG. 7B, the conductive i2i beacon message 712 does not include (i.e., is devoid of) an event marker. Further, the conductive i2i beacon message 712 does not include (i.e., is devoid of) a command code, and also does not include a payload. Thus, it should be appreciated that the conductive i2i beacon message 712 is substantially shorter than the conductive i2i communication message 702. Accordingly, transmitting an instance of the conductive i2i beacon message 712 consumes less power than transmitting an instance of the conductive i2i communication message 702, because less conductive i2i communication pulses are needed to transmit the conductive i2i beacon message 712 than to transmit the conductive i2i communication message 702. It is also within the scope of the embodiments described herein for the conductive i2i communication message 702 and/or the conductive i2i beacon message 712 to have different formats than shown, however. Regardless of the specific formats, it is preferred that the conductive i2i beacon message 712 is shorter than the conductive i2i communication message 702 so that less power is consumed to transmit an instance of the conductive i2i beacon message 712 than to transmit an instance of the conductive i2i communication message 702. Further, as explained above, when an IMD is in its second conductive i2i communication mode the IMD transmits instances of the conductive i2i beacon message 712 less often (i.e., less frequently) than the IMD transmits instances of the conductive i2i communication message 702 when the IMD is in its first conductive i2i communication mode. This is another reason that an IMD consumes less power (related to its communication capabilities) when the IMD is in its second conductive i2i communication mode compared to when the IMD is in its first conductive i2i communication mode.

While many of the embodiments of the present technology described above have been described as being for use with LP type IMDs, embodiments of the present technology, can also be used with other types of IMDs besides an LP. Accordingly, unless specifically limited to use with an LP, the claims should not be limited to use with LP type IMDs. Further, various embodiments as described in connection with FIG. 6 could be implemented separately from each other in the IMD.

FIG. 8 shows a block diagram of one embodiment of an IMD 801 (e.g., an LP 102 or ICD 106) that is configured to be implanted into the patient as part of the implantable cardiac system 100 in accordance with certain embodiments herein. IMD 801 may be implemented as a full-function biventricular pacemaker, equipped with both atrial and ventricular sensing and pacing circuitry for four chamber sensing and stimulation therapy (including both pacing and shock treatment). Optionally, IMD 801 may provide full-function cardiac resynchronization therapy. Alternatively, IMD 801 may be implemented with a reduced set of functions and components. For instance, the IMD 801 may be implemented without ventricular sensing and pacing.

IMD 801 has a housing 800 to hold the electronic/computing components. Housing 800 (which is often referred to as the "can", "case", "encasing", or "case electrode") may be programmably selected to act as the return electrode for certain stimulus modes. Housing 800 may further include a connector (not shown) with a plurality of terminals 802, 804, 806, 808, and 810. The terminals may be connected to electrodes (e.g., 108a, 108b, but not limited thereto) that are located in various locations on housing 800 or elsewhere within and about the heart. IMD 801 includes a programmable microcontroller 820 that controls various operations of IMD 801, including cardiac monitoring and stimulation therapy. Microcontroller 820 includes a microprocessor (or equivalent control circuitry), RAM and/or ROM memory, logic and timing circuitry, state machine circuitry, and I/O circuitry. The microcontroller 820, which can also be referred to herein as a controller, can be used to perform the various methods described herein, e.g., with reference to the flow diagram of FIG. 6.

IMD 801 further includes a pulse generator 822 that generates stimulation pulses for delivery by one or more electrodes coupled thereto. Pulse generator 822 is controlled by microcontroller 820 via control signal 824. Pulse generator 822 may be coupled to the select terminal(s) 802, 804, 806, 808, and 810 and thereby the select electrode(s) via an electrode configuration switch 826, which includes multiple switches for connecting the desired electrodes to the appropriate I/O circuits, thereby facilitating electrode programmability. Switch 826 is controlled by a control signal 828 from microcontroller 820.

In the embodiment of FIG. 8, a single pulse generator 822 is illustrated. Optionally, the IMD 801 may include multiple pulse generators, similar to pulse generator 822, where each pulse generator is coupled to one or more electrodes and controlled by microcontroller 820 to deliver select stimulus pulse(s) to the corresponding one or more electrodes.

Microcontroller 820 is illustrated as including timing control circuitry 832 to control the timing of the stimulation pulses (e.g., pacing rate, atrio-ventricular (AV) delay, atrial interconduction (A-A) delay, or ventricular interconduction (V-V) delay, etc.). Timing control circuitry 832 may also be used for the timing of refractory periods, blanking intervals, noise detection windows, evoked response windows, alert intervals, marker channel timing, and so on. Microcontroller 820 may also have an arrhythmia detector 834 for detecting arrhythmia conditions and optionally a morphology detector 836. Although not shown, the microcontroller 820 may further include other dedicated circuitry and/or firmware/software components that assist in monitoring various conditions of the patient's heart and managing pacing therapies.

IMD 801 is further equipped with a communication modem 840 (modulator/demodulator) to enable wireless communication with a remote pacing unit. Modem 840 may include one or more transmitters and two or more receivers as discussed herein in connection with FIG. 2. In one implementation, modem 840 may use low or high frequency modulation. As one example, modem 840 may transmit i2i messages and other signals through conductive communication between a pair of electrodes and/or using RF communication. Modem 840 may be implemented in hardware as part of microcontroller 820, or as software/firmware instructions programmed into and executed by microcontroller 820. Alternatively, modem 840 may reside separately from the microcontroller as a standalone component. In certain embodiments, the modem 840 includes both a conductive communication transceiver and an RF communication transceiver. While not specifically shown in FIG. 8, such an RF communication transceiver can be coupled to an antenna that enables RF communication signals to be transmitted and received for the purpose of transmitting and receiving messages to and from another IMD.

IMD 801 includes a sensing circuit 844 selectively coupled to one or more electrodes, that perform sensing operations, through switch 826 to detect the presence of cardiac activity in the right chambers of the heart. Sensing circuit 844 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The automatic gain control enables the unit to sense low amplitude signal characteristics of atrial fibrillation. Switch 826 determines the sensing polarity of the cardiac signal by selectively closing the appropriate switches. In this way, the clinician may program the sensing polarity independent of the stimulation polarity.

The output of sensing circuit 844 is connected to microcontroller 820 which, in turn, triggers or inhibits the pulse generator 822 in response to the presence or absence of cardiac activity. Sensing circuit 844 receives a control signal 846 from microcontroller 820 for purposes of controlling the gain, threshold, polarization charge removal circuitry (not shown), and the timing of any blocking circuitry (not shown) coupled to the inputs of the sensing circuitry.

In the embodiment of FIG. 8, a single sensing circuit 844 is illustrated. Optionally, the IMD may include multiple sensing circuits, similar to sensing circuit 844, where each sensing circuit is coupled to one or more electrodes and controlled by microcontroller 820 to sense electrical activity detected at the corresponding one or more electrodes. Sensing circuit 844 may operate in a unipolar sensing configuration or in a bipolar sensing configuration.

The IMD 801 further includes an analog-to-digital (A/D) data acquisition system (DAS) 850 coupled to one or more electrodes via switch 826 to sample cardiac signals across any pair of desired electrodes. Data acquisition system 850 is configured to acquire intracardiac electrogram signals, convert the raw analog data into digital data, and store the digital data for later processing and/or telemetric transmission to an external device 854 (e.g., a programmer, local transceiver, or a diagnostic system analyzer). Data acquisition system 850 is controlled by a control signal 856 from the microcontroller 820.

Microcontroller 820 is coupled to a memory 860 by a suitable data/address bus. The programmable operating parameters used by microcontroller 820 are stored in memory 860 and used to customize the operation of IMD 801 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart within each respective tier of therapy.

The operating parameters of IMD 801 may be non-invasively programmed into memory 860 through a telemetry circuit 864 in telemetric communication via communication link 866 with external device 854. Telemetry circuit 864 allows intracardiac electrograms and status information relating to the operation of IMD 801 (as contained in microcontroller 820 or memory 860) to be sent to external device 854 through communication link 866. Memory 860 may also store a table or other data structure that is dynamically updated over time to keep track of the types of i2i communication being used by the IMD 801 and/or other IMDs with which IMD 801 communicates.

IMD 801 can further include magnet detection circuitry (not shown), coupled to microcontroller 820, to detect when a magnet is placed over the unit. A magnet may be used by a clinician to perform various test functions of IMD 801 and/or to signal microcontroller 820 that external device 854 is in place to receive or transmit data to microcontroller 820 through telemetry circuits 864.

IMD 801 can optionally include one or more physiological sensors 870. Such sensors are commonly referred to as "rate-responsive" sensors because they are typically used to adjust pacing stimulation rates according to the exercise state of the patient. However, physiological sensor 870 may further be used to detect changes in cardiac output, changes in the physiological condition of the heart, or diurnal changes in activity (e.g., detecting sleep and wake states). Signals generated by physiological sensors 870 are passed to microcontroller 820 for analysis. Microcontroller 820 responds by adjusting the various pacing parameters (such as rate, AV Delay, V-V Delay, etc.) at which the atrial and ventricular pacing pulses are administered. While shown as being included within IMD 801, physiological sensor(s) 870 may be external to IMD 801, yet still be implanted within or carried by the patient. Examples of physiologic sensors might include sensors that, for example, sense respiration rate, pH of blood, ventricular gradient, activity, position/posture, minute ventilation (MV), and so forth.

A battery 872 provides operating power to all of the components in IMD 801. Battery 872 is capable of operating at low current drains for long periods of time, and is capable of providing high-current pulses (for capacitor charging) when the patient requires a shock pulse (e.g., in excess of 2 A, at voltages above 2 V, for periods of 10 seconds or more). Battery 872 also desirably has a predictable discharge characteristic so that elective replacement time can be detected. As one example, IMD 801 employs lithium/silver vanadium oxide batteries.

IMD 801 optionally includes an impedance measuring circuit 874, which can be used for many things, including: lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves; and so forth. Impedance measuring circuit 874 is coupled to switch 826 so that any desired electrode may be used. In this embodiment IMD 801 further includes a shocking circuit 880 coupled to microcontroller 820 by a data/address bus 882.

An aspect of the present technology relates to an IMD (e.g., 102a, 102b, 106, 801) configured to communicate using conductive i2i communication with another IMD (e.g., another one of 102a, 102b, 106, 801) that is remotely located relative to the IMD. The IMD comprises a plurality of electrodes (e.g., 108a, 108b), a conductive communication transceiver (e.g., 124), and a controller (e.g., 112, 820). The conductive communication transceiver (e.g., 124) is coupled to at least two electrodes of the plurality of electrodes (e.g., 108a, 108b) and is configured to selectively transmit outgoing conductive i2i communication messages that are intended for the other IMD (e.g., 102a, 102a, 106, 801), and configured to receive incoming conductive i2i communication messages from the other IMD (e.g., 102a, 102a, 106, 801). The controller (e.g., 112, 820) is operatively coupled to the conductive communication transceiver (e.g., 124) and configured to control when the IMD (e.g., 102a, 102b, 106, 801) is in a first conductive i2i communication mode and when the IMD (e.g., 102a, 102b, 106, 801) is in a second conductive i2i communication mode. The controller further configured to: control the conductive communication transceiver (e.g., 124) to transmit outgoing conductive i2i communication messages that includes event markers and to attempt to receive valid incoming conductive i2i communication messages that include event markers from the other IMD (e.g., 102a, 102b, 106, 801) while the IMD (e.g., 102a, 102b, 106, 801) is in the first conductive i2i communication mode; detect when a quantity of possible incoming conductive i2i communication messages that are determined to be invalid during a first specified period reach an invalid message count threshold while the IMD (e.g., 102a, 102b, 106, 801) is in the first conductive i2i communication mode, and in response thereto, cause a switch from the first conductive i2i communication mode to the second conductive i2i communication mode; and control, while the IMD (e.g., 102a, 102b, 106, 801) is in the second conductive i2i communication mode, the conductive communication transceiver (e.g., 124) to transmit outgoing conductive i2i beacon messages to the other IMD (e.g., 102a, 102b, 106, 801) less frequently than the outgoing conductive i2i communication messages transmitted when the IMD (e.g., 102a, 102b, 106, 801) is in the first conductive i2i communication mode.

In accordance with certain embodiments, the controller (e.g., 112, 820) is further configured to control the conductive communication transceiver (e.g., 124) to transmit outgoing conductive i2i communication messages that includes event markers and to attempt to receive valid incoming conductive i2i communication messages that include event markers from the other IMD (e.g., 102a, 102b, 106, 801) while the IMD (e.g., 102a, 102b, 106, 801) is in the first conductive i2i communication mode. The controller (e.g., 112, 820) is further configured to perform a message validation operation on each possible incoming conductive i2i communication message of one or more possible incoming conductive i2i communication messages received during a first specified period while the IMD (e.g., 102a, 102b, 106, 801) is in the first conductive i2i communication mode; monitor a quantity of possible incoming conductive i2i communication messages that are determined to be invalid during the first specified period while the IMD (e.g., 102a, 102b, 106, 801) is in the first conductive i2i communication mode. The controller (e.g., 112, 820) is further configured to control the conductive communication transceiver (e.g., 124) to transmit outgoing conductive i2i beacon messages to the other IMD (e.g., 102a, 102b, 106, 801) once every second specified period while the IMD (e.g., 102a, 102b, 106, 801) is in the second conductive i2i communication mode, wherein that the second specified period is longer than the first specified period, and to attempt to receive valid incoming conductive i2i beacon messages from the other IMD (e.g., 102a, 102b, 106, 801), wherein each said outgoing conductive i2i beacon message that the IMD (e.g., 102a, 102b, 106, 801) transmits is devoid of an event marker, and wherein each said valid incoming conductive i2i beacon message that the IMD (e.g., 102a, 102b, 106, 801) attempts to receive is devoid of an event marker. The controller (e.g., 112, 820) is further configured to detect when a said valid incoming conductive i2i beacon message has been received while the IMD (e.g., 102a, 102b, 106, 801) is in the second conductive i2i communication mode or determine that the IMD (e.g., 102a, 102b, 106, 801) has been in the second conductive i2i communication mode for a third specified period, and based thereon, cause a switch from the second conductive i2i communication mode back to the first conductive i2i communication mode, wherein the third specified period is longer than the second specified period.

In accordance with an embodiment, the first specified period comprises one cardiac cycle; the second specified period comprises N cardiac cycles, wherein N is a specified integer that is equal to or greater than 2; and the third specified period comprises M cardiac cycles, where M is a specified integer that is greater than N. In accordance with an embodiment, values for one or more of the invalid message count threshold, N, and M are programmable and can be changed using an external programmer.

In accordance with an embodiment, the first specified period comprises a first specified duration of time; the second specified period comprises a second specified duration of time that is longer than the first specified duration of time; and the third specified period comprises a third specified duration of time that is longer than the second specified duration of time. In accordance with an embodiment, values for one or more of the invalid message count threshold, the first specified period of time, the second specified period of time, and the third specified period of time are programmable and can be changed using an external programmer.

In accordance with an embodiment, while the IMD (e.g., 102a, 102b, 106, 801) is in the second conductive i2i communication mode and the controller (e.g., 112, 820)controls the conductive communication transceiver (e.g., 124) to attempt to receive a said valid incoming conductive i2i beacon message from the other IMD (e.g., 102a, 102b, 106, 801), the controller (e.g., 112, 820) is configured to perform the message validation operation on each possible incoming conductive i2i beacon message.

In accordance with an embodiment, each outgoing conductive i2i communication message transmitted by the conductive communication transceiver (e.g., 124) while the IMD (e.g., 102a, 102b, 106, 801) is in the first conductive i2i communication mode, each valid incoming conductive i2i communication message received by the conductive communication transceiver (e.g., 124) while the IMD (e.g., 102a, 102b, 106, 801) is in the first conductive i2i communication mode, each outgoing conductive i2i beacon message transmitted by the conductive communication transceiver (e.g., 124) while the IMD (e.g., 102a, 102b, 106, 801) is in the second conductive i2i communication mode, and each valid incoming conductive i2i beacon message received by the conductive communication transceiver (e.g., 124) while the IMD (e.g., 102a, 102b, 106, 801) is in the first conductive i2i communication mode, includes a respective error detection code that is used to perform the message validation operation.

In accordance with an embodiment, the IMD (e.g., 102a, 102b, 106, 801) comprises a first leadless pacemaker (e.g., LP) configured to be implanted in or on a first cardiac chamber and configured to deliver pacing pulses to the first cardiac chamber, and the other IMD (e.g., 102a, 102b, 106, 801) comprises a second LP configured to be implanted in or on a second cardiac chamber and configured to deliver pacing pulses to the second cardiac chamber. While the IMD (e.g., 102a, 102b, 106, 801) is in the first conductive i2i communication mode, the controller (e.g., 112, 820) is configured to time pacing pulses delivered by the first LP to the first cardiac chamber based on cardiac activity of the second cardiac chamber that the first LP learns about from event markers included in valid incoming conductive i2i communication messages received from the second LP. While the IMD (e.g., 102a, 102b, 106, 801) is in the second conductive i2i communication mode, the controller (e.g., 112, 820) is configured to operate the first LP in a safe pacing mode that does not depend on valid incoming conductive i2i communication messages being received from the second LP.

In accordance with an embodiment, the first cardiac chamber comprises an atrial chamber and the second cardiac chamber comprises a ventricular chamber, or the first cardiac chamber comprises a ventricular chamber and the second cardiac chamber comprises an atrial chamber.

In accordance with an embodiment, while the IMD (e.g., 102a, 102b, 106, 801) is in the first conductive i2i communication mode, one or more of the outgoing conductive i2i communication messages transmitted by the IMD (e.g., 102a, 102b, 106, 801) includes a payload. While the IMD (e.g., 102a, 102b, 106, 801) is in the second conductive i2i communication mode, the outgoing conductive i2i beacon messages transmitted by the IMD (e.g., 102a, 102b, 106, 801) are devoid of a payload.

In accordance with an embodiment, the second conductive i2i communication mode consumes on average at least fifty percent less power per cardiac cycle than the first conductive i2i communication mode.

In accordance with an embodiment, the controller (e.g., 112, 820) is configured to cause the IMD (e.g., 102a, 102b, 106, 801) to switch from the second conductive i2i communication mode back to the first conductive i2i communication mode in response to a said valid incoming conductive i2i beacon message being detected prior to the IMD (e.g., 102a, 102b, 106, 801) being in the second conductive i2i communication mode for the third specified period.

In accordance with another embodiment, the controller (e.g., 112, 820) is configured to cause the IMD (e.g., 102a, 102b, 106, 801) to switch from the second conductive i2i communication mode back to the first conductive i2i communication mode in response to a said valid incoming conductive i2i beacon message being detected in at least a specified number X of consecutive cardiac cycles prior to the IMD (e.g., 102a, 102b, 106, 801) being in the second conductive i2i communication mode for the third specified period, wherein X is a specified integer that is equal to or greater than 2.

In accordance with another embodiment, the controller (e.g., 112, 820) is configured to cause the IMD (e.g., 102a, 102b, 106, 801) to switch from the second conductive i2i communication mode back to the first conductive i2i communication mode in response to a said valid incoming conductive i2i beacon message being detected in at least a specified number X out of Y consecutive cardiac cycles prior to the IMD (e.g., 102a, 102b, 106, 801) being in the second conductive i2i communication mode for the third specified period, wherein X is a specified integer that is equal to or greater than 2, and Y is a specified integer that is greater than X.

In accordance with an embodiment, the controller is configured to cause the IMD (e.g., 102a, 102b, 106, 801) to switch from the second conductive i2i communication mode back to the first conductive i2i communication mode in response to the IMD (e.g., 102a, 102b, 106, 801) being in in the second conductive i2i communication mode for the third specified period without receiving a said valid incoming conductive i2i beacon message.

In accordance with an embodiment, the first conductive i2i communication mode comprises a normal conductive i2i communication mode, and the second conductive i2i communication mode comprises an i2i noise reversion with checking mode.

Another aspect of the present technology relates to a system including the IMD and the other IMD that is remotely located related to the IMD. In certain embodiments, at least one of the IMD and the other IMD is an LP. In certain such embodiments, the IMD is a first LP and the other IMD is a second LP. In certain embodiments, at least one of the IMD and the other IMD is an ICD or an ICM.

An aspect of the present technology relates to a method for use by an IMD (e.g., 102a, 102b, 106, 801) that communicates with another IMD (e.g., another one of 102a, 102b, 106, 801) using conductive i2i communication, wherein such a method can be used to improve the i2i communication between the IMDs (e.g., 102a, 102b, 106, 801). The method includes while in a first conductive i2i communication mode, the IMD (e.g., 102a, 102b, 106, 801) transmitting outgoing conductive i2i communication messages that includes event markers to the other IMD (e.g., 102a, 102b, 106, 801), and the IMD (e.g., 102a, 102b, 106, 801) attempting to receive valid incoming conductive i2i communication messages that includes event markers from the other IMD (e.g., 102a, 102b, 106, 801). The method also includes detecting when a quantity of possible incoming conductive i2i communication messages that are determined to be invalid during a first period reach an invalid message count threshold while the IMD (e.g., 102a, 102b, 106, 801) is in the first conductive i2i communication mode, and in response thereto, switching from the first conductive i2i communication mode to a second conductive i2i communication mode that consumes on average less power per cardiac cycle than the first conductive i2i communication mode. The method additionally includes while the IMD (e.g., 102a, 102b, 106, 801) is in the second conductive i2i communication mode, transmitting outgoing conductive i2i beacon messages to the other IMD (e.g., 102a, 102b, 106, 801) less frequently than the outgoing conductive i2i communication messages are transmitted when the IMD (e.g., 102a, 102b, 106, 801) is in the first conductive i2i communication mode.

In accordance with an embodiment, the method comprises while in a first conductive i2i communication mode, the IMD (e.g., 102a, 102b, 106, 801) transmitting outgoing conductive i2i communication messages that includes event markers to the other IMD (e.g., 102a, 102b, 106, 801), and the IMD (e.g., 102a, 102b, 106, 801) attempting to receive valid incoming conductive i2i communication messages that includes event markers from the other IMD (e.g., 102a, 102b, 106, 801). Additionally, the method comprises while in the first conductive i2i communication mode and while attempting to receive valid incoming conductive i2i communication messages from the other IMD (e.g., 102a, 102b, 106, 801) during, the IMD (e.g., 102a, 102b, 106, 801) performing a message validation operation on one or more possible incoming conductive i2i communication messages received during a first specified period, and the IMD (e.g., 102a, 102b, 106, 801) monitoring a quantity of possible incoming conductive i2i communication messages that are determined to be invalid during the first specified period. Additionally, the method comprises the IMD (e.g., 102a, 102b, 106, 801) detecting when the quantity of possible incoming conductive i2i communication messages that are determined to be invalid during the first specified period reach an invalid message count threshold, and in response thereto the IMD (e.g., 102a, 102b, 106, 801) switching from the first conductive i2i communication mode to a second conductive i2i communication mode that consumes on average less power per cardiac cycle than the first conductive i2i communication mode. Additionally, the method comprises while in the second conductive i2i communication mode, the IMD (e.g., 102a, 102b, 106, 801) transmitting an outgoing conductive i2i beacon message to the other IMD (e.g., 102a, 102b, 106, 801) once every second specified period, wherein the second specified period is longer the first specified period, and the IMD (e.g., 102a, 102b, 106, 801) attempting to receive valid incoming conductive i2i beacon messages from the other IMD (e.g., 102a, 102b, 106, 801), wherein each said outgoing conductive i2i beacon message that the IMD (e.g., 102a, 102b, 106, 801) transmits is devoid of an event marker, and wherein each said valid incoming conductive i2i beacon message that the IMD (e.g., 102a, 102b, 106, 801) attempts to receive is devoid of an event marker. Additionally, the method comprises while in the second conductive i2i communication mode, the IMD (e.g., 102a, 102b, 106, 801) detecting a said valid incoming conductive i2i beacon message or the IMD (e.g., 102a, 102b, 106, 801) determining that the IMD (e.g., 102a, 102b, 106, 801) has been in the second conductive i2i communication mode for a third specified period, and based thereon the IMD (e.g., 102a, 102b, 106, 801) switching from the second conductive i2i communication mode back to the first conductive i2i communication mode, wherein the third specified period is longer than the second specified period.

In accordance with an embodiment, the first specified period comprises one cardiac cycle; the second specified period comprises N cardiac cycles, wherein N is a specified integer that is equal to or greater than 2; and the third specified period comprises M cardiac cycles, where M is a specified integer that is greater than N.

In accordance with an embodiment, the first specified period comprises one cardiac cycle; the second specified period comprises N cardiac cycles, wherein N is a specified integer that is equal to or greater than 2; and the third specified period comprises M cardiac cycles, where M is a specified integer that is greater than N. In accordance with an embodiment, values for one or more of the invalid message count threshold, N, and M are programmable and can be changed using an external programmer.

In accordance with an embodiment, the first specified period comprises a first specified duration of time; the second specified period comprises a second specified duration of time that is longer than the first specified duration of time; and the third specified period comprises a third specified duration of time that is longer than the second specified duration of time. In accordance with an embodiment, values for one or more of the invalid message count threshold, the first specified period of time, the second specified period of time, and the third specified period of time are programmable and can be changed using an external programmer.

In accordance with an embodiment, the method comprises while in the second conductive i2i communication mode and while attempting to receive a said valid incoming conductive i2i beacon message from the other IMD (e.g., 102a, 102b, 106, 801), the IMD (e.g., 102a, 102b, 106, 801) performing the message validation operation on each possible incoming conductive i2i beacon message.

In accordance with an embodiment, each outgoing conductive i2i communication message transmitted by the IMD while the IMD is in the first conductive i2i communication mode, each valid incoming conductive i2i communication message received by the IMD while the IMD is in the first conductive i2i communication mode, each outgoing conductive i2i beacon message transmitted by the IMD while the IMD is in the second conductive i2i communication mode, and each valid incoming conductive i2i beacon message received by the IMD while the IMD is in the first conductive i2i communication mode, includes a respective error detection code that is used to perform the message validation operation.

In accordance with an embodiment, the IMD comprises a first LP implanted in or on a first cardiac chamber and configured to deliver pacing pulses to the first cardiac chamber, and the other IMD comprises a second LP implanted in or on a second cardiac chamber and configured to deliver pacing pulses to the second cardiac chamber. While in the first conductive i2i communication mode, timing of pacing pulses delivered by the first LP to the first cardiac chamber is based on cardiac activity of the second cardiac chamber that the first LP learns about from event markers included in valid incoming conductive i2i communication messages received from the second LP. While in the second conductive i2i communication mode, the first LP operates in a safe pacing mode that does not depend on valid incoming conductive i2i communication messages being received from the second LP.

In accordance with an embodiment, the first cardiac chamber comprises an atrial chamber and the second cardiac chamber comprises a ventricular chamber, or the first cardiac chamber comprises a ventricular chamber and the second cardiac chamber comprises an atrial chamber.

In accordance with an embodiment, while in the first conductive i2i communication mode, one or more of the outgoing conductive i2i communication messages transmitted by the IMD includes a payload; and while in the second conductive i2i communication mode, the outgoing conductive i2i beacon messages transmitted by the IMD are devoid of a payload.

In accordance with an embodiment, the second conductive i2i communication mode consumes on average at least fifty percent less power per cardiac cycle than the first conductive i2i communication mode.

In accordance with an embodiment, the method comprises the IMD detecting a said valid incoming conductive i2i beacon message prior to the IMD being in the second conductive i2i communication mode for the third specified period, and in response thereto the IMD switching from the second conductive i2i communication mode back to the first conductive i2i communication mode.

In accordance with an embodiment, the method comprises the IMD detecting a said valid incoming conductive i2i beacon message in at least a specified number X of consecutive cardiac cycles prior to the IMD being in the second conductive i2i communication mode for the third specified period, and in response thereto the IMD switching from the second conductive i2i communication mode back to the first conductive i2i communication mode, wherein X is a specified integer that is equal to or greater than 2.

In accordance with an embodiment, the method comprises the IMD detecting a said valid incoming conductive i2i beacon message in at least a specified number X out of Y consecutive cardiac cycles prior to the IMD being in the second conductive i2i communication mode for the third specified period, and in response thereto the IMD switching from the second conductive i2i communication mode back to the first conductive i2i communication mode, wherein X is a specified integer that is equal to or greater than 2, and Y is a specified integer that is greater than X.

In accordance with an embodiment, the method comprises determining when the IMD is in the second conductive i2i communication mode for the third specified duration without receiving a said valid incoming conductive i2i beacon message, and in response thereto switching the IMD from the second conductive i2i communication mode back to the first conductive i2i communication mode.

In accordance with an embodiment, the first conductive i2i communication mode comprises a normal conductive i2i communication mode; and the second conductive i2i communication mode comprises an i2i noise reversion with checking mode.

An aspect of the present technology relates to a method for use by an IMD that communicates with another IMD using conductive i2i communication, which method can be used to improve the i2i communication between the IMDs. While in a first conductive i2i communication mode, during each cardiac cycle of a plurality of cardiac cycles, the IMD transmitting an outgoing conductive i2i communication message that includes an event marker to the other IMD, and the IMD attempting to receive a valid incoming conductive i2i communication message that includes an event marker from the other IMD. While in the first conductive i2i communication mode and while attempting to receive a said valid incoming conductive i2i communication message from the other IMD during a said cardiac cycle, the IMD performing a message validation operation on each possible incoming conductive i2i communication message of one or more possible incoming conductive i2i communication messages received during the cardiac cycle, and the IMD monitoring a quantity of possible incoming conductive i2i communication messages that are determined to be invalid during the cardiac cycle. The IMD detecting that the quantity of possible incoming conductive i2i communication messages that are determined to be invalid during the cardiac cycle reach an invalid message count threshold, and in response thereto the IMD switching from the first conductive i2i communication mode to a second conductive i2i communication mode that consumes on average less power per cardiac cycle than the first conductive i2i communication mode. While in the second conductive i2i communication mode, the IMD transmitting an outgoing conductive i2i beacon message to the other IMD once every N cardiac cycles, wherein N is an integer that is equal to or greater than 2, and the IMD attempting to receive a valid incoming conductive i2i beacon message from the other IMD, wherein each said outgoing conductive i2i beacon message that the IMD transmits is devoid an event marker, and wherein the valid incoming conductive i2i beacon message that the IMD attempts to receive is devoid of an event marker. While in the second conductive i2i communication mode, the IMD detecting a said valid incoming conductive i2i beacon message or the IMD determining that the IMD has been in the second conductive i2i communication mode for M cardiac cycles, and based thereon the IMD switching from the second conductive i2i communication mode back to the first conductive i2i communication mode, wherein M is a specified integer that is greater than N.

In an embodiment, the second conductive i2i communication mode consumes on average at least fifty percent less power per cardiac cycle than the first conductive i2i communication mode.

In an embodiment, the method includes the IMD detecting a said valid incoming conductive i2i beacon message prior to the IMD being in the second conductive i2i communication mode for M cardiac cycles, and in response thereto the IMD switches from the second conductive i2i communication mode back to the first conductive i2i communication mode.

In an embodiment, the method includes the IMD detecting a said valid incoming conductive i2i beacon message in at least a specified number X of consecutive cardiac cycles prior to the IMD being in the second conductive i2i communication mode for M cardiac cycles, and in response thereto the IMD to switches from the second conductive i2i communication mode back to the first conductive i2i communication mode, wherein X is a specified integer that is equal to or greater than 2.

In an embodiment, the method includes the IMD detecting a said valid incoming conductive i2i beacon message in at least a specified number X out of Y consecutive cardiac cycles prior to the IMD being in the second conductive i2i communication mode for M cardiac cycles, and in response thereto the IMD switches from the second conductive i2i communication mode back to the first conductive i2i communication mode, wherein X is a specified integer that is equal to or greater than 2, and Y is a specified integer that is greater than X.

In an embodiment, the method includes the IMD being in in the second conductive i2i communication mode for the M cardiac cycles without receiving a said valid incoming conductive i2i beacon message, and in response thereto the IMD switches from the second conductive i2i communication mode back to the first conductive i2i communication mode.

An aspect of the present technology relates to an IMD configured to communicate using conductive i2i communication with another IMD that is remotely located relative to the IMD. The IMD comprises a plurality of electrodes, a conductive communication transceiver (e.g., e.g., 124), and a controller (e.g., 112, 820). The conductive communication transceiver (e.g., 124) is coupled to at least two of the electrodes and configured to selectively transmit conductive i2i communication messages that are intended for the other IMD, and configured to receive conductive i2i communication messages from the other IMD. The controller (e.g., 112, 820) is operatively coupled to the conductive communication transceiver (e.g., 124) and configured to control when the IMD is in a first conductive i2i communication mode and when the IMD is in a second conductive i2i communication mode. The controller (e.g., 112, 820) is further configured to control the conductive communication transceiver (e.g., 124) such that while the IMD is in the first conductive i2i communication mode, during each cardiac cycle of a plurality of cardiac cycles, an outgoing conductive i2i communication message that includes an event marker is transmitted to the other IMD, and there is an attempt to receive a valid incoming conductive i2i communication message that includes an event marker from the other IMD. The controller (e.g., 112, 820) is further configured to perform a message validation operation on each possible incoming conductive i2i communication message of one or more possible incoming conductive i2i communication messages received during the cardiac cycle while the IMD is in the first conductive i2i communication mode. The controller (e.g., 112, 820) is further configured to monitor a quantity of possible incoming conductive i2i communication messages that are determined to be invalid during the cardiac cycle while the IMD is in the first conductive i2i communication mode. The controller (e.g., 112, 820) is further configured to detect that the quantity of possible incoming conductive i2i communication messages that are determined to be invalid during the cardiac cycle reach an invalid message count threshold while the IMD is in the first conductive i2i communication mode, and in response thereto, cause a switch from the first conductive i2i communication mode to the second conductive i2i communication mode that consumes on average less power per cardiac cycle than the first conductive i2i communication mode. The controller (e.g., 112, 820) is further configured to control the conductive communication transceiver (e.g., 124) such that while the IMD is in the second conductive i2i communication mode, an outgoing conductive i2i beacon message is transmitted to the other IMD once every N cardiac cycles, wherein N is an integer that is equal to or greater than 2, and there is an attempt to receive a valid incoming conductive i2i beacon message from the other IMD, wherein each said outgoing conductive i2i beacon message that the IMD transmits is devoid an event marker, and wherein the valid incoming conductive i2i beacon message that the IMD attempts to receive is devoid of an event marker. The controller (e.g., 112, 820) is further configured to detect a said valid incoming conductive i2i beacon message has been received while the IMD is in the second conductive i2i communication mode or determine that the IMD has been in the second conductive i2i communication mode for M cardiac cycles, and based thereon, cause a switch from the second conductive i2i communication mode back to the first conductive i2i communication mode, wherein M is a specified integer that is greater than N.

In accordance with an embodiments, while the IMD is in the second conductive i2i communication mode and the controller (e.g., 112, 820) controls the conductive communication transceiver (e.g., 124) to attempt to receive a said valid incoming conductive i2i beacon message from the other IMD, the controller (e.g., 112, 820) is configured to perform the message validation operation on each possible incoming conductive i2i beacon message.

In accordance with an embodiments, each outgoing conductive i2i communication message transmitted by the conductive communication transceiver (e.g., 124) while the IMD is in the first conductive i2i communication mode, each valid incoming conductive i2i communication message received by the conductive communication transceiver (e.g., 124) while the IMD is in the first conductive i2i communication mode, each outgoing conductive i2i beacon message transmitted by the conductive communication transceiver (e.g., 124) while the IMD is in the second conductive i2i communication mode, and each valid incoming conductive i2i beacon message received by the conductive communication transceiver (e.g., 124) while the IMD is in the first conductive i2i communication mode, includes a respective error detection code that is used to perform the message validation operation.

In accordance with an embodiments, while the IMD is in the first conductive i2i communication mode, one or more of the outgoing conductive i2i communication messages transmitted by the IMD includes a payload; and while the IMD is in the second conductive i2i communication mode, the outgoing conductive i2i beacon messages transmitted by the IMD are devoid of a payload.

In accordance with an embodiments, the second conductive i2i communication mode consumes on average at least fifty percent less power per cardiac cycle than the first conductive i2i communication mode.

In accordance with an embodiments, the controller (e.g., 112, 820) is configured to cause the IMD to switch from the second conductive i2i communication mode back to the first conductive i2i communication mode in response to a said valid incoming conductive i2i beacon message being detected prior to the IMD being in the second conductive i2i communication mode for M cardiac cycles.

In accordance with another embodiments, the controller (e.g., 112, 820) is configured to cause the IMD to switch from the second conductive i2i communication mode back to the first conductive i2i communication mode in response to a said valid incoming conductive i2i beacon message being detected in at least a specified number X of consecutive cardiac cycles prior to the IMD being in the second conductive i2i communication mode for M cardiac cycles, wherein X is a specified integer that is equal to or greater than 2.

In accordance with another embodiments, the controller (e.g., 112, 820) is configured to cause the IMD to switch from the second conductive i2i communication mode back to the first conductive i2i communication mode in response to a said valid incoming conductive i2i beacon message being detected in at least a specified number X out of Y cardiac cycles prior to the IMD being in the second conductive i2i communication mode for M cardiac cycles, wherein X is a specified integer that is equal to or greater than 2, and Y is a specified integer that is greater than X.

In accordance with an embodiments, the controller (e.g., 112, 820) is configured to cause the IMD to switch from the second conductive i2i communication mode back to the first conductive i2i communication mode in response to the IMD being in in the second conductive i2i communication mode for the M cardiac cycles without receiving a said valid incoming conductive i2i beacon message.

An aspect of the present technology is related to a system comprising a first IMD and a second IMD configured to communicate with one another using conductive i2i communication. The first IMD is configured to: operate in a first conductive i2i communication mode and in a second conductive i2i communication mode; transmit an outgoing conductive i2i communication message that includes an event marker to the second IMD, and attempt to receive a valid incoming conductive i2i communication message that includes an event marker from the second IMD, during each cardiac cycle of a plurality of cardiac cycles, while the first IMD operates in the first conductive i2i communication mode; and perform a message validation operation on each possible incoming conductive i2i communication message of one or more possible incoming conductive i2i communication messages received during a specified period, and monitor a quantity of possible incoming conductive i2i communication messages that are determined to be invalid during the specified period, while the first IMD operates in the first conductive i2i communication mode and attempts to receive a said valid incoming conductive i2i communication message from the second IMD during the cardiac cycle. The first IMD is also configured to detect that the quantity of possible incoming conductive i2i communication messages that are determined to be invalid during the specified period reach an invalid message count threshold, and in response thereto switch from the first conductive i2i communication mode to the second conductive i2i communication mode that consumes on average less power per cardiac cycle than the first conductive i2i communication mode. The first IMD is also configured to transmit an outgoing conductive i2i beacon message to the second IMD once every N cardiac cycles, and attempt to receive a valid incoming conductive i2i beacon message from the second IMD, while in the second conductive i2i communication mode, wherein N is an integer that is equal to or greater than 2, wherein each said outgoing conductive i2i beacon message that the first IMD transmits is devoid an event marker, and wherein the valid incoming conductive i2i beacon message that the first IMD attempts to receive is devoid of an event marker. Additionally, the first IMD is configured to switch from the second conductive i2i communication mode back to the first conductive i2i communication mode, based on a said valid incoming conductive i2i beacon message being detected or the first IMD being in the second conductive i2i communication mode for M cardiac cycles, wherein M is a specified integer that is greater than N.

In accordance with an embodiment, the first conductive i2i communication mode comprises a normal conductive i2i communication mode; and the second conductive i2i communication mode comprises an i2i noise reversion with checking mode.

An aspect of the present technology is related to a method for use by an IMD that communicates with another IMD using conductive i2i communication, to improve the i2i communication between the IMDs. The method comprises: while in a first conductive i2i communication mode, the IMD transmitting outgoing conductive i2i communication messages that includes an event marker to the other IMD, and the IMD attempting to receive a valid incoming conductive i2i communication message that includes an event marker from the other IMD; the IMD detecting that a quantity of possible incoming conductive i2i communication messages that are determined to be invalid during a specified period reach an invalid message count threshold, and in response thereto the IMD switching from the first conductive i2i communication mode to a second conductive i2i communication mode that consumes on average less power per cardiac cycle than the first conductive i2i communication mode. While in the second conductive i2i communication mode, the IMD transmitting outgoing conductive i2i beacon messages to the other IMD less frequently than the IMD transmits outgoing conductive i2i communication messages when the IMD is in the first conductive i2i communication mode.

In accordance with an embodiment, each of the outgoing conductive i2i beacon messages that the IMD transmits while in the second conductive i2i communication mode is shorter than each of the outgoing conductive i2i communication messages that the IMD transmits while in the first conductive i2i communication mode.

In accordance with an embodiment, the method further comprises while in the second conductive i2i communication mode, the IMD attempting to receive a valid incoming conductive i2i beacon message from the other IMD.

In accordance with an embodiment, the method further comprises while in the second conductive i2i communication mode, the IMD detecting a said valid incoming conductive i2i beacon message or the IMD determining that the IMD has been in the second conductive i2i communication mode for M cardiac cycles, and based thereon the IMD switching from the second conductive i2i communication mode back to the first conductive i2i communication mode.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

The scope of the embodiments of the present technology should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An implantable medical device, IMD, (102a, 102b, 106, 801) configured to communicate using conductive implant-to-implant, i2i, communication with another IMD (102a, 102b, 106, 801) that is remotely located relative to the IMD (102a, 102b, 106, 801), the IMD (102a, 102b, 106, 801) comprising:
a plurality of electrodes (108a, 108b);
a conductive communication transceiver (124) coupled to at least two electrodes (108a, 108b) of the plurality of electrodes (108a, 108b) and configured to selectively transmit outgoing conductive i2i communication messages (702) that are intended for the other IMD (102a, 102b, 106, 801), and configured to receive incoming conductive i2i communication messages (702) from the other IMD (102a, 102b, 106, 801);
a controller (112, 820) operatively coupled to the conductive communication transceiver (124) and configured to control when the IMD (102a, 102b, 106, 801) is in a first conductive i2i communication mode and when the IMD (102a, 102b, 106, 801) is in a second conductive i2i communication mode that consumes on average less power per cardiac cycle than the first conductive i2i communication mode;
the controller (112, 820) further configured to
control the conductive communication transceiver (124) to transmit outgoing conductive i2i communication messages (702) that includes event markers (704) and to attempt to receive valid incoming conductive i2i communication messages (702) that include event markers (704) from the other IMD (102a, 102b, 106, 801) while the IMD (102a, 102b, 106, 801) is in the first conductive i2i communication mode;
detect when a quantity of possible incoming conductive i2i communication messages that are determined to be invalid during a first specified period reach an invalid message count threshold while the IMD (102a, 102b, 106, 801) is in the first conductive i2i communication mode, and in response thereto, cause a switch from the first conductive i2i communication mode to the second conductive i2i communication mode; and
control, while the IMD (102a, 102b, 106, 801) is in the second conductive i2i communication mode, the conductive communication transceiver (124) to transmit outgoing conductive i2i beacon messages (712) to the other IMD (102a, 102b, 106, 801) less frequently than the outgoing conductive i2i communication messages (702) transmitted when the IMD (102a, 102b, 106, 801) is in the first conductive i2i communication mode.

2. The IMD (102a, 102b, 106, 801) of claim 1, wherein the controller (112, 820) is further configured to:
perform a message validation operation on each possible incoming conductive i2i communication message of one or more possible incoming conductive i2i communication messages received during the first specified period while the IMD (102a, 102b, 106, 801) is in the first conductive i2i communication mode;
monitor the quantity of possible incoming conductive i2i communication messages that are determined to be invalid during the first specified period while the IMD (102a, 102b, 106, 801) is in the first conductive i2i communication mode;
control the conductive communication transceiver (124) to transmit outgoing conductive i2i beacon messages (712) to the other IMD (102a, 102b, 106, 801) once every second specified period while the IMD (102a, 102b, 106, 801) is in the second conductive i2i communication mode, wherein the second specified period is longer than the first specified period, and to attempt to receive valid incoming conductive i2i beacon messages from the other IMD (102a, 102b, 106, 801), wherein each said outgoing conductive i2i beacon message (712) that the IMD (102a, 102b, 106, 801) transmits is devoid of an event marker (704), and wherein each said valid incoming conductive i2i beacon message (712) that the IMD (102a, 102b, 106, 801) attempts to receive is devoid of an event marker (704); and
detect when a said valid incoming conductive i2i beacon message (712) has been received while the IMD (102a, 102b, 106, 801) is in the second conductive i2i communication mode or determine that the IMD (102a, 102b, 106, 801) has been in the second conductive i2i communication mode for a third specified period, and based thereon, cause a switch from the second conductive i2i communication mode back to the first conductive i2i communication mode, wherein the third specified period is longer than the second specified period.

3. The IMD (102a, 102b, 106, 801) of claim 2, wherein:
the first specified period comprises one cardiac cycle;
the second specified period comprises N cardiac cycles, wherein N is a specified integer that is equal to or greater than 2; and
the third specified period comprises M cardiac cycles, where M is a specified integer that is greater than N.

4. The IMD (102a, 102b, 106, 801) of claim 3, wherein values for one or more of the invalid message count threshold, N, and M are programmable and can be changed using an external programmer.

5. The IMD (102a, 102b, 106, 801) of claim 2, wherein:
the first specified period comprises a first specified duration of time;
the second specified period comprises a second specified duration of time that is longer than the first specified duration of time; and
the third specified period comprises a third specified duration of time that is longer than the second specified duration of time.

6. The IMD (102a, 102b, 106, 801) of any one of claims 2 through 5, wherein while the IMD (102a, 102b, 106, 801) is in the second conductive i2i communication mode and the controller (112, 820) controls the conductive communication transceiver (124) to attempt to receive a said valid incoming conductive i2i beacon message (712) from the other IMD (102a, 102b, 106, 801) , the controller (112, 820) is configured to perform the message validation operation on each possible incoming conductive i2i beacon message.

7. The IMD (102a, 102b, 106, 801) of claim 6, wherein each outgoing conductive i2i communication message (702) transmitted by the conductive communication transceiver (124) while the IMD (102a, 102b, 106, 801) is in the first conductive i2i communication mode, each valid incoming conductive i2i communication message (702) received by the conductive communication transceiver (124) while the IMD (102a, 102b, 106, 801) is in the first conductive i2i communication mode, each outgoing conductive i2i beacon message (712) transmitted by the conductive communication transceiver (124) while the IMD (102a, 102b, 106, 801) is in the second conductive i2i communication mode, and each valid incoming conductive i2i beacon message (712) received by the conductive communication transceiver (124) while the IMD (102a, 102b, 106, 801) is in the first conductive i2i communication mode, includes a respective error detection code (710, 716) that is used to perform the message validation operation.

8. The IMD (102a, 102b, 106, 801) of any one of claims 2 through 7, wherein the controller (112, 820) is configured to cause the IMD (102a, 102b, 106, 801) to switch from the second conductive i2i communication mode back to the first conductive i2i communication mode in response to a said valid incoming conductive i2i beacon message (712) being detected prior to the IMD (102a, 102b, 106, 801) being in the second conductive i2i communication mode for the third specified period.

9. The IMD (102a, 102b, 106, 801) of any one of claims 2 through 7, wherein the controller (112, 820) is configured to cause the IMD (102a, 102b, 106, 801) to switch from the second conductive i2i communication mode back to the first conductive i2i communication mode in response to a said valid incoming conductive i2i beacon message (712) being detected in at least a specified number X of consecutive cardiac cycles prior to the IMD (102a, 102b, 106, 801) being in the second conductive i2i communication mode for the third specified period, wherein X is a specified integer that is equal to or greater than 2.

10. The IMD (102a, 102b, 106, 801) of any one of claims 2 through 7, wherein the controller (112, 820) is configured to cause the IMD (102a, 102b, 106, 801) to switch from the second conductive i2i communication mode back to the first conductive i2i communication mode in response to a said valid incoming conductive i2i beacon message (712) being detected in at least a specified number X out of Y consecutive cardiac cycles prior to the IMD (102a, 102b, 106, 801) being in the second conductive i2i communication mode for the third specified period, wherein X is a specified integer that is equal to or greater than 2, and Y is a specified integer that is greater than X.

11. The IMD (102a, 102b, 106, 801) of any one of claims 1 through 10, wherein:
the IMD (102a, 102b, 106, 801) comprises a first leadless pacemaker, LP, (102a, 102b) configured to be implanted in or on a first cardiac chamber and configured to deliver pacing pulses to the first cardiac chamber;
the other IMD (102a, 102b, 106, 801) comprises a second LP (102a, 102b) configured to be implanted in or on a second cardiac chamber and configured to deliver pacing pulses to the second cardiac chamber;
while the IMD (102a, 102b, 106, 801) is in the first conductive i2i communication mode, the controller (112, 820) is configured to time pacing pulses delivered by the first LP to the first cardiac chamber based on cardiac activity of the second cardiac chamber that the first LP learns about from event markers included in valid incoming conductive i2i communication messages received from the second LP; and
while the IMD (102a, 102b, 106, 801) is in the second conductive i2i communication mode, the controller (112, 820) is configured to operate the first LP (102a, 102b) in a safe pacing mode that does not depend on valid incoming conductive i2i communication messages being received from the second LP (102a, 102b).

12. The IMD (102a, 102b, 106, 801) of any one of claims 1 through 11, wherein:
while the IMD (102a, 102b, 106, 801) is in the first conductive i2i communication mode, one or more of the outgoing conductive i2i communication messages (702) transmitted by the IMD (102a, 102b, 106, 801) includes a payload (708); and
while the IMD (102a, 102b, 106, 801) is in the second conductive i2i communication mode, the outgoing conductive i2i beacon messages (712) transmitted by the IMD (102a, 102b, 106, 801) are devoid of a payload (708).

13. The IMD (102a, 102b, 106, 801) of any one of claims 1 through 12, wherein:
the second conductive i2i communication mode consumes on average at least fifty percent less power per cardiac cycle than the first conductive i2i communication mode.

14. The IMD (102a, 102b, 106, 801) of any one of claims 1 through 13, wherein:
the first conductive i2i communication mode comprises a default conductive i2i communication mode during which the IMD (102a, 102b, 106, 801) transmits a conductive i2i communication message including an event marker to the other IMD (102a, 102b, 106, 801) once per cardiac cycle so that the other IMD (102a, 102b, 106, 801) can time its pacing and/or other functions based on the event marker the other IMD (102a, 102b, 106, 801) receives from the IMD (102a, 102b, 106, 801); and
the second conductive i2i communication mode comprises an i2i noise reversion with checking mode.

## Patentansprüche

1. Implantierbare medizinische Vorrichtung, IMD, (102a, 102b, 106, 801), die dazu konfiguriert ist, unter Verwendung einer leitfähigen Implantat-zu-Implantat-Kommunikation, i2i-Kommunikation, mit einer anderen IMD (102a, 102b, 106, 801) zu kommunizieren, die sich in Bezug auf die IMD (102a, 102b, 106, 801) entfernt befindet, wobei die IMD (102a, 102b, 106, 801) Folgendes umfasst:
eine Vielzahl von Elektroden (108a, 108b);
einen Sendeempfänger (124) für leitfähige Kommunikation, der mit mindestens zwei Elektroden (108a, 108b) der Vielzahl von Elektroden (108a, 108b) gekoppelt und dazu konfiguriert ist, ausgehende leitfähige i2i-Kommunikationsnachrichten (702), die für die andere IMD (102a, 102b, 106, 801) bestimmt sind, selektiv zu übertragen, und dazu konfiguriert ist, eingehende leitfähige i2i-Kommunikationsnachrichten (702) von der anderen IMD (102a, 102b, 106, 801) zu empfangen;
eine Steuerung (112, 820), die betriebswirksam an den Sendeempfänger (124) für leitfähige Kommunikation gekoppelt und dazu konfiguriert ist, zu steuern, wann sich die IMD (102a, 102b, 106, 801) in einem ersten leitfähigen i2i-Kommunikationsmodus befindet und wann sich die IMD (102a, 102b, 106, 801) in einem zweiten leitfähigen i2i-Kommunikationsmodus befindet, der durchschnittlich weniger Leistung pro Herzzyklus verbraucht als der erste leitfähige i2i-Kommunikationsmodus;
wobei die Steuerung (112, 820) ferner zu Folgendem konfiguriert ist:
Steuern des Sendeempfängers (124) für leitfähige Kommunikation, um ausgehende leitfähige i2i-Kommunikationsnachrichten (702), die Ereignismarker (704) beinhaltet, zu übertragen und zu versuchen, gültige eingehende leitfähige i2i-Kommunikationsnachrichten (702), die Ereignismarker (704) beinhalten, von der anderen IMD (102a, 102b, 106, 801) zu empfangen, während sich die IMD (102a, 102b, 106, 801) in dem ersten leitfähigen i2i-Kommunikationsmodus befindet;
Detektieren, wann eine Menge möglicher eingehender leitfähiger i2i-Kommunikationsnachrichten, die während eines ersten spezifizierten Zeitraums als ungültig bestimmt werden, einen ungültigen Nachrichtenzählschwellenwert erreicht, während sich die IMD (102a, 102b, 106, 801) in dem ersten leitfähigen i2i-Kommunikationsmodus befindet, und als Reaktion darauf eine Umschaltung von dem ersten leitfähigen i2i-Kommunikationsmodus in den zweiten leitfähigen i2i-Kommunikationsmodus zu veranlassen; und
Steuern, während sich die IMD (102a, 102b, 106, 801) in dem zweiten leitfähigen i2i-Kommunikationsmodus befindet, des Sendeempfängers (124) für leitfähige Kommunikation, um ausgehende leitfähige i2i-Beacon-Nachrichten (712) weniger häufig an die andere IMD (102a, 102b, 106, 801) zu übertragen als die ausgehenden leitfähigen i2i-Kommunikationsnachrichten (702), die übertragen werden, wenn sich die IMD (102a, 102b, 106, 801) in dem ersten leitfähigen i2i-Kommunikationsmodus befindet.

2. IMD (102a, 102b, 106, 801) nach Anspruch 1, wobei die Steuerung (112, 820) ferner zu Folgendem konfiguriert ist:
Durchführen eines Nachrichtenvalidierungsvorgangs an jeder möglichen eingehenden leitfähigen i2i-Kommunikationsnachricht einer oder mehrerer möglicher eingehender leitfähiger i2i-Kommunikationsnachrichten, die während des ersten spezifizierten Zeitraums empfangen werden, während sich die IMD (102a, 102b, 106, 801) in dem ersten leitfähigen i2i-Kommunikationsmodus befindet;
Überwachen der Menge möglicher eingehender leitfähiger i2i-Kommunikationsnachrichten, die während des ersten spezifizierten Zeitraums als ungültig bestimmt werden, während sich die IMD (102a, 102b, 106, 801) in dem ersten leitfähigen i2i-Kommunikationsmodus befindet;
Steuern des Sendeempfängers (124) für leitfähige Kommunikation, um ausgehende leitfähige i2i-Beacon-Nachrichten (712) jeden zweiten spezifizierten Zeitraum einmal an die andere IMD (102a, 102b, 106, 801) zu übertragen, während sich die IMD (102a, 102b, 106, 801) in dem zweiten leitfähigen i2i-Kommunikationsmodus befindet, wobei der zweite spezifizierte Zeitraum länger als der erste spezifizierte Zeitraum ist, und zu versuchen, gültige eingehende leitfähige i2i-Beacon-Nachrichten von der anderen IMD (102a, 102b, 106, 801) zu empfangen, wobei jede der ausgehenden leitfähigen i2i-Beacon-Nachrichten (712), welche die IMD (102a, 102b, 106, 801) überträgt, frei von einem Ereignismarker (704) ist und wobei jede der gültigen eingehenden leitfähigen i2i-Beacon-Nachrichten (712), welche die IMD (102a, 102b, 106, 801) zu empfangen versucht, frei von einem Ereignismarker (704) ist; und
Detektieren, wann eine der gültigen eingehenden leitfähigen i2i-Beacon-Nachrichten (712) empfangen wurde, während sich die IMD (102a, 102b, 106, 801) in dem zweiten leitfähigen i2i-Kommunikationsmodus befindet, oder Bestimmen, dass sich die IMD (102a, 102b, 106, 801) für einen dritten spezifizierten Zeitraum in dem zweiten leitfähigen i2i-Kommunikationsmodus befand, und darauf basierend eine Umschaltung von dem zweiten leitfähigen i2i-Kommunikationsmodus zurück in den ersten leitfähigen i2i-Kommunikationsmodus zu veranlassen, wobei der dritte spezifizierte Zeitraum länger als der zweite spezifizierte Zeitraum ist.

3. IMD (102a, 102b, 106, 801) nach Anspruch 2, wobei:
der erste spezifizierte Zeitraum einen Herzzyklus umfasst;
der zweite spezifizierte Zeitraum N Herzzyklen umfasst, wobei N eine spezifizierte ganze Zahl ist, die gleich oder größer als 2 ist; und
der dritte spezifizierte Zeitraum M Herzzyklen umfasst, wobei M eine spezifizierte ganze Zahl ist, die größer als N ist.

4. IMD (102a, 102b, 106, 801) nach Anspruch 3, wobei Werte für eines oder mehrere des ungültigen Nachrichtenzählschwellenwerts, N und M programmierbar sind und unter Verwendung eines externen Programmierers geändert werden können.

5. IMD (102a, 102b, 106, 801) nach Anspruch 2, wobei:
der erste spezifizierte Zeitraum eine erste spezifizierte Zeitdauer umfasst;
der zweite spezifizierte Zeitraum eine zweite spezifizierte Zeitdauer umfasst, die länger als die erste spezifizierte Zeitdauer ist; und
der dritte spezifizierte Zeitraum eine dritte spezifizierte Zeitdauer umfasst, die länger als die zweite spezifizierte Zeitdauer ist.

6. IMD (102a, 102b, 106, 801) nach einem der Ansprüche 2 bis einschließlich 5, wobei, während sich die IMD (102a, 102b, 106, 801) in dem zweiten leitfähigen i2i-Kommunikationsmodus befindet und die Steuerung (112, 820) den Sendeempfänger (124) für leitfähige Kommunikation steuert, um zu versuchen, eine der gültigen eingehenden leitfähigen i2i-Beacon-Nachrichten (712) von der anderen IMD (102a, 102b, 106, 801) zu empfangen, die Steuerung (112, 820) dazu konfiguriert ist, den Nachrichtenvalidierungsvorgang an jeder möglichen eingehenden leitfähigen i2i-Beacon-Nachricht durchzuführen.

7. IMD (102a, 102b, 106, 801) nach Anspruch 6, wobei jede ausgehende leitfähige i2i-Kommunikationsnachricht (702), die durch den Sendeempfänger (124) für leitfähige Kommunikation übertragen wird, während sich die IMD (102a, 102b, 106, 801) in dem ersten leitfähigen i2i-Kommunikationsmodus befindet, jede gültige eingehende leitfähige i2i-Kommunikationsnachricht (702), die durch den Sendeempfänger (124) für leitfähige Kommunikation empfangen wird, während sich die IMD (102a, 102b, 106, 801) in dem ersten leitfähigen i2i-Kommunikationsmodus befindet, jede ausgehende leitfähige i2i-Beacon-Nachricht (712), die durch den Sendeempfänger (124) für leitfähige Kommunikation übertragen wird, während sich die IMD (102a, 102b, 106, 801) in dem zweiten leitfähigen i2i-Kommunikationsmodus befindet, und jede gültige eingehende leitfähige i2i-Beacon-Nachricht (712), die durch den Sendeempfänger (124) für leitfähige Kommunikation empfangen wird, während sich die IMD (102a, 102b, 106, 801) in dem ersten leitfähigen i2i-Kommunikationsmodus befindet, einen jeweiligen Fehlerdetektionscode (710, 716) beinhaltet, der dazu verwendet wird, den Nachrichtenvalidierungsvorgang durchzuführen.

8. IMD (102a, 102b, 106, 801) nach einem der Ansprüche 2 bis einschließlich 7, wobei die Steuerung (112, 820) dazu konfiguriert ist, als Reaktion darauf, dass eine der gültigen eingehenden leitfähigen i2i-Beacon-Nachrichten (712) detektiert wird, bevor sich die IMD (102a, 102b, 106, 801) für den dritten spezifizierten Zeitraum in dem zweiten leitfähigen i2i-Kommunikationsmodus befindet, die IMD (102a, 102b, 106, 801) dazu zu veranlassen, von dem zweiten leitfähigen i2i-Kommunikationsmodus zurück in den ersten leitfähigen i2i-Kommunikationsmodus umzuschalten.

9. IMD (102a, 102b, 106, 801) nach einem der Ansprüche 2 bis einschließlkich 7, wobei die Steuerung (112, 820) dazu konfiguriert ist, als Reaktion darauf, dass eine der gültigen eingehenden leitfähigen i2i-Beacon-Nachrichten (712) in mindestens einer spezifizierten Anzahl X aufeinanderfolgender Herzzyklen detektiert wird, bevor sich die IMD (102a, 102b, 106, 801) für den dritten spezifizierten Zeitraum in dem zweiten leitfähigen i2i-Kommunikationsmodus befindet, die IMD (102a, 102b, 106, 801) dazu zu veranlassen, von dem zweiten leitfähigen i2i-Kommunikationsmodus zurück in den ersten leitfähigen i2i-Kommunikationsmodus umzuschalten, wobei X eine spezifizierte ganze Zahl ist, die gleich oder größer als 2 ist.

10. IMD (102a, 102b, 106, 801) nach einem der Ansprüche 2 bis einschließlich 7, wobei die Steuerung (112, 820) dazu konfiguriert ist, als Reaktion darauf, dass eine der gültigen eingehenden leitfähigen i2i-Beacon-Nachrichten (712) in mindestens einer spezifizierten Anzahl X aus Y aufeinanderfolgenden Herzzyklen detektiert wird, bevor sich die IMD (102a, 102b, 106, 801) für den dritten spezifizierten Zeitraum in dem zweiten leitfähigen i2i-Kommunikationsmodus befindet, die IMD (102a, 102b, 106, 801) dazu zu veranlassen, von dem zweiten leitfähigen i2i-Kommunikationsmodus zurück in den ersten leitfähigen i2i-Kommunikationsmodus umzuschalten, wobei X eine spezifizierte ganze Zahl ist, die gleich oder größer als 2 ist und Y eine spezifizierte ganze Zahl ist, die größer als X ist.

11. IMD (102a, 102b, 106, 801) nach einem der Ansprüche 1 bis einschließlich 10, wobei:
die IMD (102a, 102b, 106, 801) einen ersten leitungslosen Schrittmacher, LP, (102a, 102b) umfasst, der dazu konfiguriert ist, in oder an einer ersten Herzkammer implantiert zu sein, und dazu konfiguriert ist, Schrittmachungsimpulse an die erste Herzkammer abzugeben;
die andere IMD (102a, 102b, 106, 801) einen zweiten LP (102a, 102b) umfasst, der dazu konfiguriert ist, in oder an einer zweiten Herzkammer implantiert zu sein, und dazu konfiguriert ist, Schrittmachungsimpulse an die zweite Herzkammer abzugeben;
während sich die IMD (102a, 102b, 106, 801) in dem ersten leitfähigen i2i-Kommunikationsmodus befindet, die Steuerung (112, 820) dazu konfiguriert ist, Schrittmachungsimpulse, die durch den ersten LP an die erste Herzkammer abgegeben werden, basierend auf einer Herzaktivität der zweiten Herzkammer zeitlich zu planen, über die der erste LP aus Ereignismarkern lernt, die in gültigen eingehenden leitfähigen i2i-Kommunikationsnachrichten beinhaltet sind, die von dem zweiten LP empfangen werden; und
während sich die IMD (102a, 102b, 106, 801) in dem zweiten leitfähigen i2i-Kommunikationsmodus befindet, die Steuerung (112, 820) dazu konfiguriert ist, den ersten LP (102a, 102b) in einem sicheren Schrittmachungsmodus zu betreiben, der nicht davon abhängt, dass gültige eingehende leitfähige i2i-Kommunikationsnachrichten von dem zweiten LP (102a, 102b) empfangen werden.

12. IMD (102a, 102b, 106, 801) nach einem der Ansprüche 1 bis einschließlich 11, wobei:
während sich die IMD (102a, 102b, 106, 801) in dem ersten leitfähigen i2i-Kommunikationsmodus befindet, eine oder mehrere der ausgehenden leitfähigen i2i-Kommunikationsnachrichten (702), die durch die IMD (102a, 102b, 106, 801) übertragen werden, eine Nutzlast (708) beinhalten; und
während sich die IMD (102a, 102b, 106, 801) in dem zweiten leitfähigen i2i-Kommunikationsmodus befindet, die ausgehenden leitfähigen i2i-Beacon-Nachrichten (712), die durch die IMD (102a, 102b, 106, 801) übertragen werden, frei von einer Nutzlast (708) sind.

13. IMD (102a, 102b, 106, 801) nach einem der Ansprüche 1 bis einschließlich 12, wobei:
der zweite leitfähige i2i-Kommunikationsmodus durchschnittlich mindestens fünfzig Prozent weniger Leistung pro Herzzyklus verbraucht als der erste leitfähige i2i-Kommunikationsmodus.

14. IMD (102a, 102b, 106, 801) nach einem der Ansprüche 1 bis einschließlich 13, wobei:
der erste leitfähige i2i-Kommunikationsmodus einen standardmäßigen leitfähigen i2i-Kommunikationsmodus umfasst, während dessen die IMD (102a, 102b, 106, 801) eine leitfähige i2i-Kommunikationsnachricht, die einen Ereignismarker beinhaltet, einmal pro Herzzyklus an die andere IMD (102a, 102b, 106, 801) überträgt, sodass die andere IMD (102a, 102b, 106, 801) ihre Schrittmachungs- und/oder anderen Funktionen basierend auf dem Ereignismarker, den die andere IMD (102a, 102b, 106, 801) von der IMD (102a, 102b, 106, 801) empfängt, zeitlich planen kann; und
der zweite leitfähige i2i-Kommunikationsmodus eine i2i-Rauschen-Reversion mit Prüfmodus umfasst.

## Revendications

1. Dispositif médical implantable, DMI, (102a, 102b, 106, 801) configuré pour communiquer en utilisant une communication d'implant à implant, i2i, conductrice avec un autre DMI (102a, 102b, 106, 801) qui est situé à distance par rapport au DMI (102a, 102b, 106, 801), le DMI (102a, 102b, 106, 801) comprenant :
une pluralité d'électrodes (108a, 108b) ;
un émetteur-récepteur de communication conductrice (124) couplé à au moins deux électrodes (108a, 108b) de la pluralité d'électrodes (108a, 108b) et configuré pour transmettre sélectivement des messages de communication i2i conductrice sortants (702) qui sont destinés à l'autre DMI (102a, 102b, 106, 801), et configuré pour recevoir des messages de communication i2i conductrice entrants (702) provenant de l'autre DMI (102a, 102b, 106, 801) ;
un dispositif de commande (112, 820) couplé de manière fonctionnelle à l'émetteur-récepteur de communication conductrice (124) et configuré pour commander le moment où le DMI (102a, 102b, 106, 801) est dans un premier mode de communication i2i conductrice et le moment où le DMI (102a, 102b, 106, 801) est dans un second mode de communication i2i conductrice qui consomme en moyenne moins d'énergie par cycle cardiaque que le premier mode de communication i2i conductrice ; le dispositif de commande (112, 820) étant en outre configuré pour commander à l'émetteur-récepteur de communication conductrice (124) de transmettre des messages de communication i2i conductrice sortants (702) qui inclut des marqueurs d'événement (704) et de tenter de recevoir des messages de communication i2i conductrice entrants valides (702) qui incluent des marqueurs d'événement (704) provenant de l'autre DMI (102a, 102b, 106, 801) pendant que le DMI (102a, 102b, 106, 801) est dans le premier mode de communication i2i conductrice ; détecter le moment où une quantité de messages de communication i2i conductrice entrants possibles qui sont déterminés comme étant invalides pendant une première période spécifiée atteignent un seuil de comptage de messages invalides pendant que le DMI (102a, 102b, 106, 801) est dans le premier mode de communication i2i conductrice, et en réponse à cela, provoquer un passage du premier mode de communication i2i conductrice au second mode de communication i2i conductrice ; et
commander, pendant que le DMI (102a, 102b, 106, 801) est dans le second mode de communication i2i conductrice, à l'émetteur-récepteur de communication conductrice (124) de transmettre des messages de balise i2i conductrice sortants (712) à l'autre DMI (102a, 102b, 106, 801) moins fréquemment que les messages de communication i2i conductrice sortants (702) transmis lorsque le DMI (102a, 102b, 106, 801) est dans le premier mode de communication i2i conductrice.

2. DMI (102a, 102b, 106, 801) selon la revendication 1, dans lequel le dispositif de commande (112, 820) est en outre configuré pour :
réaliser une opération de validation de message sur chaque message de communication i2i conductrice entrant possible d'un ou plusieurs messages de communication i2i conductrice entrants possibles reçus pendant la première période spécifiée pendant que le DMI (102a, 102b, 106, 801) est dans le premier mode de communication i2i conductrice ;
surveiller la quantité de messages de communication i2i conductrice entrants possibles qui sont déterminés comme étant invalides pendant la première période spécifiée pendant que le DMI (102a, 102b, 106, 801) est dans le premier mode de communication i2i conductrice ;
commander à l'émetteur-récepteur de communication conductrice (124) de transmettre des messages de balise i2i conductrice sortants (712) à l'autre DMI (102a, 102b, 106, 801) une fois chaque deuxième période spécifiée pendant que le DMI (102a, 102b, 106, 801) est dans le second mode de communication i2i conductrice, dans lequel la deuxième période spécifiée est plus longue que la première période spécifiée, et de tenter de recevoir des messages de balise i2i conductrice entrants valides provenant de l'autre DMI (102a, 102b, 106, 801), dans lequel chaque dit message de balise i2i conductrice sortant (712) que le DMI (102a, 102b, 106, 801) transmet est dépourvu d'un marqueur d'événement (704), et dans lequel chaque dit message de balise i2i conductrice entrant valide (712) que le DMI (102a, 102b, 106, 801) tente de recevoir est dépourvu d'un marqueur d'événement (704) ; et
détecter le moment où un dit message de balise i2i conductrice entrant valide (712) a été reçu pendant que le DMI (102a, 102b, 106, 801) est dans le second mode de communication i2i conductrice ou déterminer que le DMI (102a, 102b, 106, 801) a été dans le second mode de communication i2i conductrice pendant une troisième période spécifiée, et sur la base de cela, provoquer un passage du second mode de communication i2i conductrice de retour au premier mode de communication i2i conductrice, dans lequel la troisième période spécifiée est plus longue que la deuxième période spécifiée.

3. DMI (102a, 102b, 106, 801) selon la revendication 2, dans lequel :
la première période spécifiée comprend un cycle cardiaque ;
la deuxième période spécifiée comprend N cycles cardiaques, dans lequel N est un entier spécifié qui est égal ou supérieur à 2 ; et
la troisième période spécifiée comprend M cycles cardiaques, où M est un entier spécifié qui est supérieur à N.

4. DMI (102a, 102b, 106, 801) selon la revendication 3, dans lequel des valeurs pour l'un ou plusieurs parmi le seuil de comptage de messages invalides, N et M sont programmables et peuvent être modifiées en utilisant un programmateur externe.

5. DMI (102a, 102b, 106, 801) selon la revendication 2, dans lequel :
la première période spécifiée comprend une première durée spécifiée ;
la deuxième période spécifiée comprend une deuxième durée spécifiée qui est plus longue que la première durée spécifiée ; et
la troisième période spécifiée comprend une troisième durée spécifiée qui est plus longue que la deuxième durée spécifiée.

6. DMI (102a, 102b, 106, 801) selon l'une quelconque des revendications 2 à 5, dans lequel pendant que le DMI (102a, 102b, 106, 801) est dans le second mode de communication i2i conductrice et que le dispositif de commande (112, 820) commande à l'émetteur-récepteur de communication conductrice (124) de tenter de recevoir un dit message de balise i2i conductrice entrant valide (712) provenant de l'autre DMI (102a, 102b, 106, 801), le dispositif de commande (112, 820) est configuré pour réaliser l'opération de validation de message sur chaque message de balise i2i conductrice entrant possible.

7. DMI (102a, 102b, 106, 801) selon la revendication 6, dans lequel chaque message de communication i2i conductrice sortant (702) transmis par l'émetteur-récepteur de communication conductrice (124) pendant que le DMI (102a, 102b, 106, 801) est dans le premier mode de communication i2i conductrice, chaque message de communication i2i conductrice entrant valide (702) reçu par l'émetteur-récepteur de communication conductrice (124) pendant que le DMI (102a, 102b, 106, 801) est dans le premier mode de communication i2i conductrice, chaque message de balise i2i conductrice sortant (712) transmis par l'émetteur-récepteur de communication conductrice (124) pendant que le DMI (102a, 102b, 106, 801) est dans le second mode de communication i2i conductrice, et chaque message de balise i2i conductrice entrant valide (712) reçu par l'émetteur-récepteur de communication conductrice (124) pendant que le DMI (102a, 102b, 106, 801) est dans le premier mode de communication i2i conductrice, inclut un code de détection d'erreur respectif (710, 716) qui est utilisé pour réaliser l'opération de validation de message.

8. DMI (102a, 102b, 106, 801) selon l'une quelconque des revendications 2 à 7, dans lequel le dispositif de commande (112, 820) est configuré pour amener le DMI (102a, 102b, 106, 801) à passer du second mode de communication i2i conductrice de retour au premier mode de communication i2i conductrice en réponse à un dit message de balise i2i conductrice entrant valide (712) étant détecté avant que le DMI (102a, 102b, 106, 801) ne soit dans le second mode de communication i2i conductrice pendant la troisième période spécifiée.

9. DMI (102a, 102b, 106, 801) selon l'une quelconque des revendications 2 à 7, dans lequel le dispositif de commande (112, 820) est configuré pour amener le DMI (102a, 102b, 106, 801) à passer du second mode de communication i2i conductrice de retour au premier mode de communication i2i conductrice en réponse à un dit message de balise i2i conductrice entrant valide (712) étant détecté dans au moins un nombre spécifié X de cycles cardiaques consécutifs avant que le DMI (102a, 102b, 106, 801) ne soit dans le second mode de communication i2i conductrice pendant la troisième période spécifiée, dans lequel X est un entier spécifié qui est égal ou supérieur à 2.

10. DMI (102a, 102b, 106, 801) selon l'une quelconque des revendications 2 à 7, dans lequel le dispositif de commande (112, 820) est configuré pour amener le DMI (102a, 102b, 106, 801) à passer du second mode de communication i2i conductrice de retour au premier mode de communication i2i conductrice en réponse à un dit message de balise i2i conductrice entrant valide (712) étant détecté dans au moins un nombre spécifié X sur Y cycles cardiaques consécutifs avant que le DMI (102a, 102b, 106, 801) ne soit dans le second mode de communication i2i conductrice pendant la troisième période spécifiée, dans lequel X est un entier spécifié qui est égal ou supérieur à 2, et Y est un entier spécifié qui est supérieur à X.

11. DMI (102a, 102b, 106, 801) selon l'une quelconque des revendications 1 à 10, dans lequel :
le DMI (102a, 102b, 106, 801) comprend un premier stimulateur cardiaque sans sonde, SCSS, (102a, 102b) configuré pour être implanté dans ou sur une première cavité cardiaque et configuré pour délivrer des impulsions de stimulation à la première cavité cardiaque ;
l'autre DMI (102a, 102b, 106, 801) comprend un second SCSS (102a, 102b) configuré pour être implanté dans ou sur une seconde cavité cardiaque et configuré pour délivrer des impulsions de stimulation à la seconde cavité cardiaque ;
pendant que le DMI (102a, 102b, 106, 801) est dans le premier mode de communication i2i conductrice, le dispositif de commande (112, 820) est configuré pour minuter des impulsions de stimulation délivrées par le premier SCSS à la première cavité cardiaque sur la base d'une activité cardiaque de la seconde cavité cardiaque dont le premier SCSS prend connaissance à partir de marqueurs d'événement inclus dans des messages de communication i2i conductrice entrants valides reçus en provenance du second SCSS ; et
pendant que le DMI (102a, 102b, 106, 801) est dans le second mode de communication i2i conductrice, le dispositif de commande (112, 820) est configuré pour faire fonctionner le premier SCSS (102a, 102b) dans un mode de stimulation de sécurité qui ne dépend pas de messages de communication i2i conductrice entrants valides étant reçus en provenance du second SCSS (102a, 102b).

12. DMI (102a, 102b, 106, 801) selon l'une quelconque des revendications 1 à 11, dans lequel :
pendant que le DMI (102a, 102b, 106, 801) est dans le premier mode de communication i2i conductrice, l'un ou plusieurs parmi les messages de communication i2i conductrice sortants (702) transmis par le DMI (102a, 102b, 106, 801) incluent une charge utile (708) ; et
pendant que le DMI (102a, 102b, 106, 801) est dans le second mode de communication i2i conductrice, les messages de balise i2i conductrice sortants (712) transmis par le DMI (102a, 102b, 106, 801) sont dépourvus d'une charge utile (708).

13. DMI (102a, 102b, 106, 801) selon l'une quelconque des revendications 1 à 12, dans lequel :
le second mode de communication i2i conductrice consomme en moyenne au moins cinquante pour cent d'énergie en moins par cycle cardiaque que le premier mode de communication i2i conductrice.

14. DMI (102a, 102b, 106, 801) selon l'une quelconque des revendications 1 à 13, dans lequel :
le premier mode de communication i2i conductrice comprend un mode de communication i2i conductrice par défaut pendant lequel le DMI (102a, 102b, 106, 801) transmet un message de communication i2i conductrice incluant un marqueur d'événement à l'autre DMI (102a, 102b, 106, 801) une fois par cycle cardiaque de sorte que l'autre DMI (102a, 102b, 106, 801) puisse minuter sa stimulation et/ou d'autres fonctions sur la base du marqueur d'événement que l'autre DMI (102a, 102b, 106, 801) reçoit en provenance du DMI (102a, 102b, 106, 801) ; et
le second mode de communication i2i conductrice comprend un mode de réversion de bruit i2i avec vérification.
